(19) Europäisches Patentamt
European Patent Office
Office européen des brevets

(11) EP 1 729 818 B1

(12) **FASCICULE DE BREVET EUROPEEN**

(45) Date de publication et mention de la délivrance du brevet:
**23.09.2009 Bulletin 2009/39**

(21) Numéro de dépôt: **05746947.0**

(22) Date de dépôt: **31.03.2005**

(51) Int Cl.:
*A61K 49/00* (2006.01) *A61K 49/06* (2006.01)
*A61K 49/12* (2006.01)

(86) Numéro de dépôt international:
**PCT/FR2005/000784**

(87) Numéro de publication internationale:
**WO 2005/094903 (13.10.2005 Gazette 2005/41)**

(54) **AGENTS DE CONTRASTE POUR L'IMAGERIE PAR RESONANCE MAGNETIQUE**

KONTRASTMITTEL FÜR DIE MAGNETRESONANZTOMOGRAPHIE

CONTRAST AGENTS FOR MAGNETIC RESONANCE IMAGING

(84) Etats contractants désignés:
**AT BE BG CH CY CZ DE DK EE ES FI FR GB GR HU IE IS IT LI LT LU MC NL PL PT RO SE SI SK TR**

(30) Priorité: **31.03.2004 FR 0403389**

(43) Date de publication de la demande:
**13.12.2006 Bulletin 2006/50**

(73) Titulaires:
- **Centre National de la Recherche Scientifique (CNRS)
75016 Paris (FR)**
- **Ecole Normale Superieure Sciences De Lyon
69364 Lyon Cedex 07 (FR)**

(72) Inventeur: **HASSERODT, Jens
F-69006 LYON (FR)**

(74) Mandataire: **Desaix, Anne et al
Ernest Gutmann - Yves Plasseraud S.A.S.
3, rue Auber
75009 Paris (FR)**

(56) Documents cités:
**US-A- 5 707 605**

- **WIEGHARDT K ET AL: "SYNTHESES, PROPERTIES, AND ELECTROCHEMISTRY OF TRANSITION-METAL COMPLEXES OF THE MACROCYLE 1,4,7-TRIS(2-PYRIDYLMETHYL)-1,4,7- TRIAZACYCLONONANE (L). CRYSTAL STRUCTURES DISTORTED-SQUARE-BASE-PYRAMIDAL PDIIN5 CORE" INORGANIC CHEMISTRY, AMERICAN CHEMICAL SOCIETY, EASTON, US, vol. 25, no. 27, 1986, pages 4877-4883, XP001004830 ISSN: 0020-1669 cité dans la demande**
- **CHRISTIANSEN, LISE ET AL: "Synthesis and structure of metal complexes of triaza macrocycles with three pendant pyridylmethyl arms" INORGANIC CHEMISTRY , 25(16), 2813-18 CODEN: INOCAJ; ISSN: 0020-1669, 1986, XP002304972**
- **TSUKUBE, HIROSHI ET AL: "Triazamacrocycle having pyridine-pendant arms as a new sodium ion-selective ionophore" TETRAHEDRON LETTERS , 30(30), 3983-6 CODEN: TELEAY; ISSN: 0040-4039, 1989, XP002304971**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne des agents de contraste pour l'imagerie par résonance magnétique.

L'imagerie par résonance magnétique (IRM) est une technique largement utilisée dans le domaine médical, notamment aux fins de diagnostic. L'IRM repose principalement sur l'observation de la magnétisation des spins des noyaux des atomes d'hydrogène dans les molécules d'eau. Elle consiste à mesurer la relaxation par deux modes (T1 et T2) des molécules d'eau par rapport à une distribution à l'équilibre, pour former une image. La relaxation survient après un état dans lequel tous les spins ont été complètement alignés sous l'effet d'un champ magnétique extérieur par utilisation d'une impulsion de radiofréquence. L'IRM permet de distinguer entre les zones de forte concentration et les zones de faible concentration d'un agent de contraste. Dans le domaine biologique, l'image obtenue met en évidence les tissus riches en agent de contraste, essentiellement ceux qui sont hydrophiles par nature.

Les agents de contraste de la première génération sont des agents dits "passifs" qui sont répartis à travers l'échantillon soumis à l'IRM, essentiellement en fonction des gradients de lipophilicité et des liaisons non spécifiques.

Des travaux ont été effectués pour définir des agents actifs, c'est-à-dire des agents capables de révéler la présence d'une activité chimique ou biochimique, de conditions de pH ou une concentration d'un ion particulier. Ces agents sont constitués par un ion fortement paramagnétique complexé par un ligand, l'ion étant généralement Gd(III). Les agents de contraste contenant un tel ion fortement paramagnétique constituent par conséquent un aimant moléculaire permanent, qui exerce toujours une influence, plus ou moins importante, sur la relaxation des molécules d'eau environnantes. Le moyen pour rendre ces complexes de type aimant moléculaire permanent sensibles à des composés chimiques spécifiques de leur environnement reposait essentiellement sur la modification de l'accessibilité de l'environnement immédiat du métal paramagnétique pour les molécules d'eau. De tels marqueurs, pour lesquels la relaxation par une activité enzymatique est améliorée par le fait qu'ils agissent comme catalyseurs pour l'échange rapide de molécules d'eau, sont décrits dans divers documents.

US-5,707,605 décrit un agent IRM qui est un complexe d'un ion de métal paramagnétique et d'un ligand, ledit ligand comprenant un groupe chélatant et un substituant qui est lié par liaison covalente au chélatant. En l'absence de substance cible, le substituant est associé ou lié à l'ion métallique du complexe, et il occupe ou bloque au moins un site de coordination de l'ion métallique. Tous les sites de coordination de l'ion métallique sont alors saturés par le chélatant et le substituant. En présence de la substance cible, le substituant réagit avec ladite substance et cesse de bloquer ou d'occuper au moins un site de coordination de l'ion métallique, de sorte qu'un échange rapide d'eau peut s'effectuer sur le site, ce qui donne une amélioration de l'image RM. Un dérivé de l'acide 1,4,7,10-tétraazacyclododécane-N,N', N",N"'-tétraacétique (DOTA) dans lequel l'un des substituant acétate est remplacé par un substituant hydroxyéthyl β-galactose est un exemple d'un tel complexe. Dans ce complexe, le groupe terminal sensible à l'enzyme n'a qu'un faible effet protecteur du centre métallique vis à vis de la coordination par l'eau : le nombre moyen de molécules d'eau qui sont coordinées à tout moment au 9$^{ème}$ site de coordination du gadolinium est de 0,65 au lieu de 1,02 pour la forme "clivée" d'un composé similaire contenant un ligand capable d'occuper seulement 8 sites de coordination. (Cf. Meade, T. J. et al. "In vivo visualization of gene expression using magnetic resonance imaging. Nature Biotechnology 18, 321-325 (2000*)").* Ce faible accès supplémentaire des molécules d'eau durant la bioactivation de l'agent de contraste ne donne pas une modification satisfaisante de la relaxation.

En outre, la demanderesse a relevé un autre inconvénient lié à cet agent de contraste, à savoir que celui-ci ne réagit pas avec une vitesse suffisante avec la substance cible, à savoir l'enzyme béta-galactosidase. En effet, la demanderesse a mené des expériences qui ont montré qu'une co-injection de ce même agent de contraste avec l'enzyme dans un organisme vivant de test, à savoir des embryons du poisson zèbre, ne conduit pas à une quelconque modification du contraste, ni après 24 heures, ni après 48 heures. Ce résultat a été obtenu en comparaison avec une expérience identique dans laquelle l'agent co-injecté était un agent chromogénique commercial qui a donné lieu à une coloration intense de l'embryon liée à sa transformation enzymatique.

WO 99/21592 décrit l'application de la technique de US-5,707,605 à une substance cible du type agent thérapeutique. L'amélioration du taux de contraste qui peut être obtenue à l'aide des marqueurs du type de ceux décrits dans les deux documents précités est cependant limitée.

On connaît des complexes formés par un ion de métal de transition et par un ligand qui peuvent exister sous une forme haut spin et sous une forme bas spin suivant la nature du ligand. A l'état haut spin, la quasi-totalité des électrons sont des électrons impairs, il n'y a pas de compensation interne de leur spins, ce qui produit une augmentation substantielle du moment magnétique. A l'état bas spin, les complexes ont un moment magnétique faible, voire nul, suivant le nombre d'électrons impairs (en général 0 ou 1). Un exemple de composé de Fe(II) complexé par un ligand macrocyclique hexadentate à spin faible est donné par Wieghardt et al. (Inorg. Chem. 1986, 25, 4877) et un exemple de composé de Fe(II) complexé par un ligand macrocyclique pentadentate similaire à spin élevé est donné par Spiccia et al. (Inorg. Chim. Acta, 1998, 279, 192).

Le but de l'invention est de fournir un agent de contraste capable de détecter une substance cible ou l'activité chimique associée à cette substance cible dans une cellule, ou un tissu soumis à l'IRM, durant la phase d'acquisition

et de construction de l'image.

Un agent de contraste pour l'IRM selon la présente invention est un complexe comprenant un ligand chélatant et un ion de métal de transition, ledit ligand portant un substituant capable de réagir par voie chimique ou biochimique avec une substance cible en provoquant un changement de l'état de spin et possédant :

a) soit la formule (I)

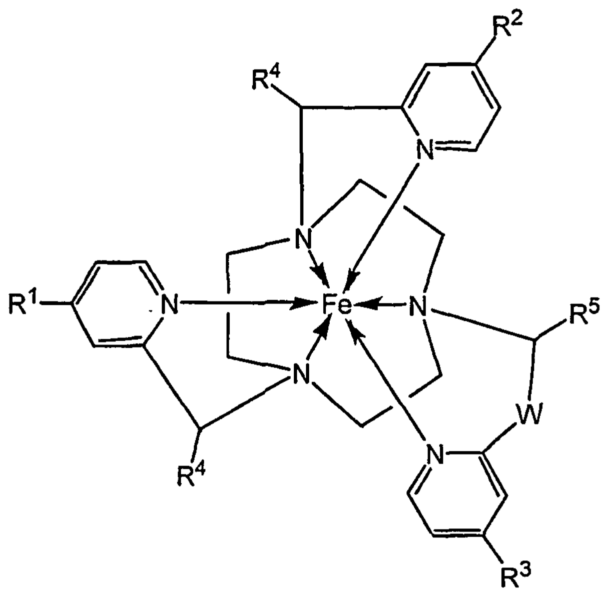

I

dans laquelle :

- $R^4$ représente un atome d'hydrogène ou un groupe alkyle de 1 à 4 atomes de carbone, linéaire ou ramifié, ou un radical aryle ;
- W représente un groupe $-(CH_2)_m-$, m étant égal à 0 ou à 1 ;
- $R^5$ représente un groupe capable de réagir avec la substance cible pour provoquer une coupure entre l'atome N du macrocycle et le carbone portant ledit groupe $R^5$, ou représente un atome d'hydrogène, étant entendu que :

    i) lorsque $R^5$ représente un groupe capable de réagir avec la substance cible pour provoquer une coupure entre l'atome N du macrocycle et le carbone portant ledit groupe $R^5$ (complexes Ia), $R^1$ à $R^3$, désignés par $R^i$, représentent, indépendamment les uns des autres, un atome d'hydrogène ou un groupe choisi pour ajuster les propriétés de solubilité, de dispersibilité dans les milieux biologiques, et de moment magnétique du complexe, l'un des $R^i$ pouvant encore représenter un groupe capable de subir une modification électronique du groupement pyridyle voisin par réaction avec la substance cible;

    ii)lorsque $R^5$ représente un atome d'hydrogène (complexes Ib), l'un des groupes $R^i$ représente un groupe capable de subir une modification électronique du groupement pyridyle voisin par réaction avec la substance cible, les groupes $R^i$ restant pouvant représenter, indépendamment les uns des autres, un atome d'hydrogène ou être choisis pour ajuster les propriétés de solubilité, de dispersibilité dans les milieux biologiques, et de moment magnétique du complexe ;

b) soit la formule (II)

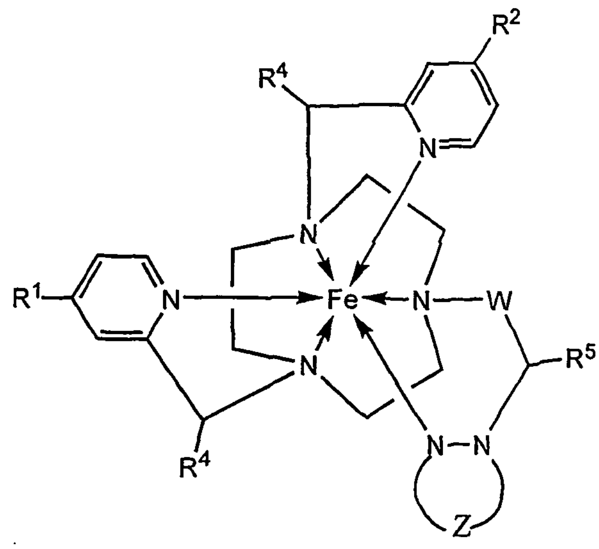

dans laquelle :

- $R^1$ et $R^2$, désignés par $R^i$, représentent, indépendamment les uns des autres, un atome d'hydrogène ou un groupe choisi pour ajuster les propriétés de solubilité, de dispersibilité dans les milieux biologiques, et de moment magnétique du complexe ;
- $R^4$ et W sont tels que définis précédemment ;
- $R^5$ représente un groupe ($-C_6X_4-Y-R^5$) dans lequel X est un groupement électroattracteur, Y est un hétéroatome choisi parmi un atome d'oxygène, un atome d'azote ou un atome de soufre, et $R^6$ représente un groupe β-galactosyle ou un groupe β-glucuronyle susceptible de subir un clivage par une glycosidase, ou un groupe aminoacyle (par exemple L-prolyle ou *L*-leucyle) susceptible de subir un clivage par une aminopeptidase, ou un groupe acyle, tel que le $-COC_5H_{11}$, susceptible de subir un clivage par une esterase ou une lipase, ou un groupe susceptible de subir une transformation retro- aldol par une aldolase naturelle, par exemple un groupe de type α,β-dihydroxycétone

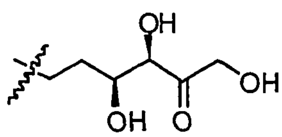,

ou un groupe phosphoryle susceptible de subir une transformation par une phosphatase ;
- Z est un groupement divalent qui forme avec les deux atomes d'azote qui le portent un cycle aromatique benzotriazole, triazole, tétrazole, pyrazole, ledit cycle aromatique pouvant porter un substituant $NO_2$.

Dans un composé (II), les groupes X peuvent représenter, indépendamment les uns des autres, un atome d'hydrogène, un atome de fluor, ou un groupe $NO_2$.

Les groupes $R^4$ sont choisis pour augmenter la rigidité du ligand, et, par conséquent, pour augmenter la stabilité du complexe métallique initial et du complexe final après transformation par la substance cible.

Le choix du groupe W est fait pour affiner la vitesse d'auto-effondrement.

Le changement de l'état de spin est obtenu par l'élimination ou par la modification électronique d'un substituant.

Lorsqu'il s'agit de l'élimination d'un substituant, le changement de spin est un passage d'un bas spin (complexe diamagnétique et inactif en RM) à un spin élevé (complexe paramagnétique et actif en RM).

Lorsqu'il s'agit de la modification électronique d'un substituant, le changement de spin est généralement un passage d'un bas spin à un haut spin. Cependant, un changement dans le sens contraire peut également être obtenu par le choix d'un ligand approprié.

Le métal de transition du composé de formule (I) ou (II) est le Fer, pour lequel un changement d'état d'oxydation de

II à III est préférable. En effet, le Fer(III) haut-spin est reconnu pour son pouvoir élevé par rapport au Fer (II) haut-spin de modifier la relaxation des protons voisins, grâce à son temps de relaxation électronique $\tau_s$ plus élevé : $10^{-9}$-$10^{-11}$ s (Fe(III) haut-spin) ; $10^{-12}$-$10^{-13}$ s (Fe(II) haut-spin)(voir les publications de I. Bertini, C. Luchinat, G. Parigi : « Solution NMR of Paramagnetic Molecules », Current Methods in Inorganic Chemistry Vol.2, Elsevier, Amsterdam 2001 ; et R. B. Lauffer, Chem. Rev. 1987, 87, 901). Des composés à base de Fe(II) haut-spin ont souvent tendance à être oxydés pour atteindre l'état électronique $d^5$ du Fer(III) qui constitue un état très stable grâce à une couche électronique *d* demi-remplie. Par contre, un Fer(III) entouré par un ligand multi-denté fournissant un champ fort, comme c'est le cas dans cette invention, conduit à un placement de l'ensemble de ses 6 électrons en état apparié dans des orbitales d de basse énergie (3 orbitales dégénérées de type $t_2g$), ce qui stabilise cet état d'oxydation, car il n'y a pas de possibilité d'enlever un électron de haute énergie lors de l'oxydation, comme c'est le cas pour le Fer (II) haut-spin. C'est donc un avantage considérable de cette invention de partir d'un traceur stable à l'oxydation et sans spin électronique, pour arriver, après initiation enzymatique, à un complexe de spin élevé possédant un temps de relaxation électronique favorable à l'imagerie RMN. Le spin électronique du Fer (III) haut-spin « communique bien » avec le spin nucléaire d'un proton.

Dans le cadre de la présente invention

- un atome d'halogène signifie, un atome de chlore, de fluor, de brome ou d'iode. D'une manière générale, on préfère l'atome de chlore et de fluor ;
- un « cycle aromatique à 5 ou 6 chaînons » signifie un groupe éventuellement hétérocyclique choisi parmi un groupe phényle, pyridyle, pyrimidinyle, pyrazinyle, indoyle, indazolyle, furyle et thiényle et leurs dérivées portant différents substituants choisis parmi les groupes méthyle, chloro, fluoro, nitro, méthoxy, carboxy, et acétamido ;
- un « cycle aliphatique à 5 ou 6 chaînons » signifie un groupe cyclohexyle ou un groupe cyclopentyle ;
- un groupe « alkyle » signifie une chaîne aliphatique linéaire ou ramifiée de 1 à 4 atomes de carbone ;
- un « radical aryle » signifie un groupe comprenant un ou plusieurs noyaux aromatiques condensés ou non.

Selon un premier mode de réalisation des complexes de formule I, le substituant $R^5$ est un groupe capable de réagir avec la substance cible pour provoquer une coupure entre l'atome N du macrocycle et le carbone portant ledit groupe $R^5$. Ces complexes particuliers sont désignés par Ia.

Dans les complexes Ia, les groupes $R^i$ représentent, indépendamment les uns des autres, un atome d'hydrogène, un groupe électroattracteur choisi parmi le groupe $-COOR^7$, le groupe $-NO_2$ et un atome d'halogène afin de contrôler l'état de spin du métal, ou un groupe portant des charges négatives tel que le groupe $-CH_2-COO^-$ pour compenser les deux charges positives du Fer(II) et donc rendre l'agent globalement neutre.

L'un des trois groupes $R^i$ peut en outre représenter un groupe $-CH(OH)CH(COOH)NH_2$, les $R^i$ restant étant alors tels que définis ci-dessus.

$R^7$ peut représenter un groupe alkyle ayant de 1 à 4 atomes de carbone. Un tel groupe $R^7$ peut être utile pour réduire la polarité de l'agent entier.

Selon une première variante des complexes Ia, désignée ci-après par Ia-1, $R^5$ peut être représenté par la formule $-E-R^6$ dans laquelle $R^6$ est tel que défini ci-dessus et E est un groupe espaceur capable de s'auto-effondrer en cas de coupure de la liaison $E-R^6$. $R^6$ est capable de réagir avec un composé chimique ou un composé biochimique en déclenchant une succession de coupures provoquant l'élimination de la pyridine-2-carbaldéhyde, selon le mécanisme exposé dans la description ci-après.

E peut être un groupe $-O-CO-(CR'_2)_n-Y-$ dans lequel :

- les groupes R' représentent, indépendamment les uns des autres, un atome d'hydrogène, ou un groupe choisi pour préorganiser l'espaceur afin d'accélérer son auto-effondrement après clivage du $R^6$. Plus particulièrement, les groupes R' peuvent représenter un groupe méthyle, ou deux R' portés par deux atomes de carbone voisins forment ensemble un cycle aliphatique ou aromatique à 5 ou 6 chaînons porté par les deux atomes de carbone voisins (voir par exemple : L.A. Carpino., J. Org. Chem. 1989, 54, 3303) ;
- n est un entier égal à 3 ou 4,
- Y est un hétéroatome choisi parmi un atome d'oxygène, un atome d'azote ou un atome de soufre.

Le cycle aromatique à 5 ou 6 chaînons formé par deux groupes R' peut être choisi parmi les groupes phényle, pyridyle, pyrimidinyle, pyrazinyle, indoyle, indazolyle, furyle et thiényle, lesdits groupes pouvant porter différents substituants choisis parmi les groupes méthyle, chloro, fluoro, nitro, méthoxy, carboxy et acétamido. Le cycle aliphatique à 5 ou 6 chaînons formé par deux groupes R' peut être un groupe cyclohexyle ou un groupe cyclopentyle.

Comme exemple de groupe E dans un composé Ia-I, on peut citer les groupes suivants :

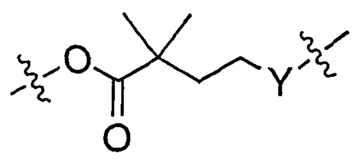 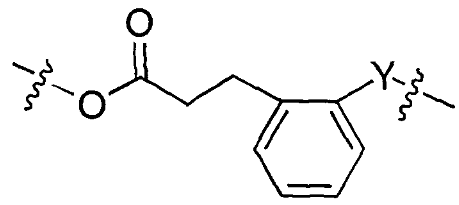

E1 E2

Dans un composé Ia-I, l'élimination de la pyridine-2-carbaldéhyde forme un complexe ayant un ligand pentadentate, ce qui change l'état de spin du complexe et en même temps permet la fixation d'une molécule d'eau sur un site de coordination du fer.
Selon une deuxième variante des complexes Ia, désignée ci-après par Ia-2, $R^5$ peut être représenté par un groupe $C_6X_4$-Y-$R^6$ dans lequel ($C_6X_4$-Y) est un groupe espaceur capable de s'auto-effondrer en cas de coupure de la liaison ($C_6X_4$-y)-$R^6$ et $R^6$ est un groupe réagissant avec un composé chimique ou un composé biochimique en déclenchant une succession de coupures provoquant l'élimination d'une quinone-méthide, selon le mécanisme exposé dans la description ci-après.

Plus particulièrement, X, Y et $R^6$ ont la signification donnée précédemment.

Dans un complexe Ia-2, le groupe ($C_6X_4$-Y) peut prendre l'une des significations qui suivent :

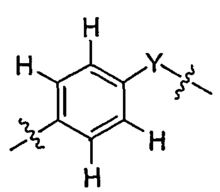 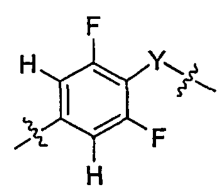 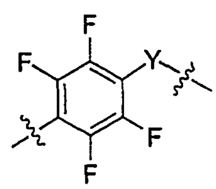 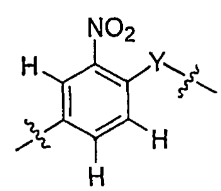

L'élimination de la quinone-méthide forme un complexe ayant un ligand pentadentate, ce qui change l'état de spin du complexe et en même temps permet la fixation d'une molécule d'eau sur un site de coordination du fer.

Selon un deuxième mode de réalisation des complexes de formule I, le substituant $R^5$ représente un atome d'hydrogène. Ces complexes sont désignés ci-après par Ib.

Dans les complexes Ib, l'un des groupes $R^i$ représente un groupe capable de subir une modification électronique du groupement pyridyle voisin par réaction avec la substance cible, par exemple un groupe -CH(OH)CH(COOH)$NH_2$, et les groupes $R^i$ restant peuvent représenter, indépendamment les uns des autres, un atome d'hydrogène, un atome d'halogène, ou un groupe choisi parmi les groupes -$COOR^7$ dans lesquels $R^7$ peut représenter un groupe alkyle ayant de 1 à 4 atomes de carbone, le groupe -$NO_2$, le groupe -CHO.

Un complexe de type (Ib) change d'état de spin sous l'effet d'une modification de l'un des substituants $R^i$. Par exemple, un substituant $R^i$ du type -CH(OH)CH(COOH)$NH_2$ se réorganise sous l'action de la L-thréonine aldolase, en produisant un changement de l'état de spin. Aucun accès n'est offert à une molécule d'eau dans la première sphère de coordination lors de sa transformation en -CHO, selon le mécanisme exposé ci-après. Seules les molécules d'eau de la deuxième sphère de coordination sont influencées.

Selon un mode de réalisation des complexes de formule générale II, les groupes X peuvent représenter, indépendamment les uns des autres, un atome d'hydrogène, un atome de fluor, ou un groupe $NO_2$, et le groupe ($C_6X_4$-Y) peut prendre l'une des significations qui suivent :

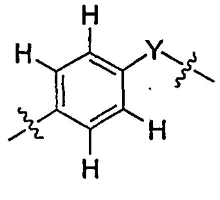 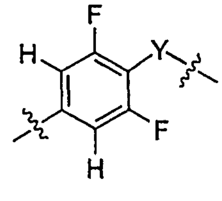 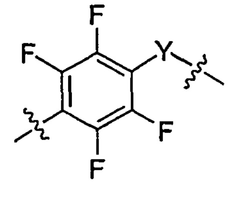 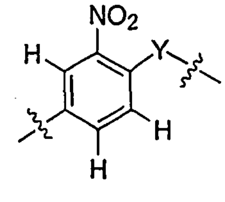

Un complexe selon l'invention peut être utilisé comme marqueur en IRM, pour détecter une substance cible telle que par exemple une enzyme.

L'action d'un complexe du type (Ia-I) est illustrée par le schéma réactionnel (schéma 1) ci-dessous, pour un complexe dans lequel $R^5$ est un groupe -E-$R^6$, E est un groupe espaceur - O-CO-C$(CH_3)_2$-$(CH_2)_2$-O-, $R^6$ est un groupe β-galactosyle, les groupes $R^4$ représentent des atomes d'hydrogène et W est une simple liaison (autrement dit m=0). Ce type de complexe permet la détection de β-galactosidase. Lorsque le complexe (Ia-1) est mis en présence de β-galactosidase, il se produit une coupure du groupe β-galactosyle avec formation de β-galactose, puis une coupure spontanée du groupe espaceur avec formation de la lactone 4,4-diméthyle-dihydro-furan-2-one, puis une coupure spontanée entre l'atome de carbone qui portait le substituant $R^5$ et l'atome d'azote du macrocycle, avec formation de pyridine-2-carbaldéhyde. Une molécule d'eau peut alors se fixer sur le site de coordination libéré, et le centre de Fer(II) adopte l'état haut-spin. Ce composé s'oxyde spontanément vers l'état d'oxydation III pour devenir un agent de contraste en IRM de haute efficacité.

**Schéma 1**

(Ia)

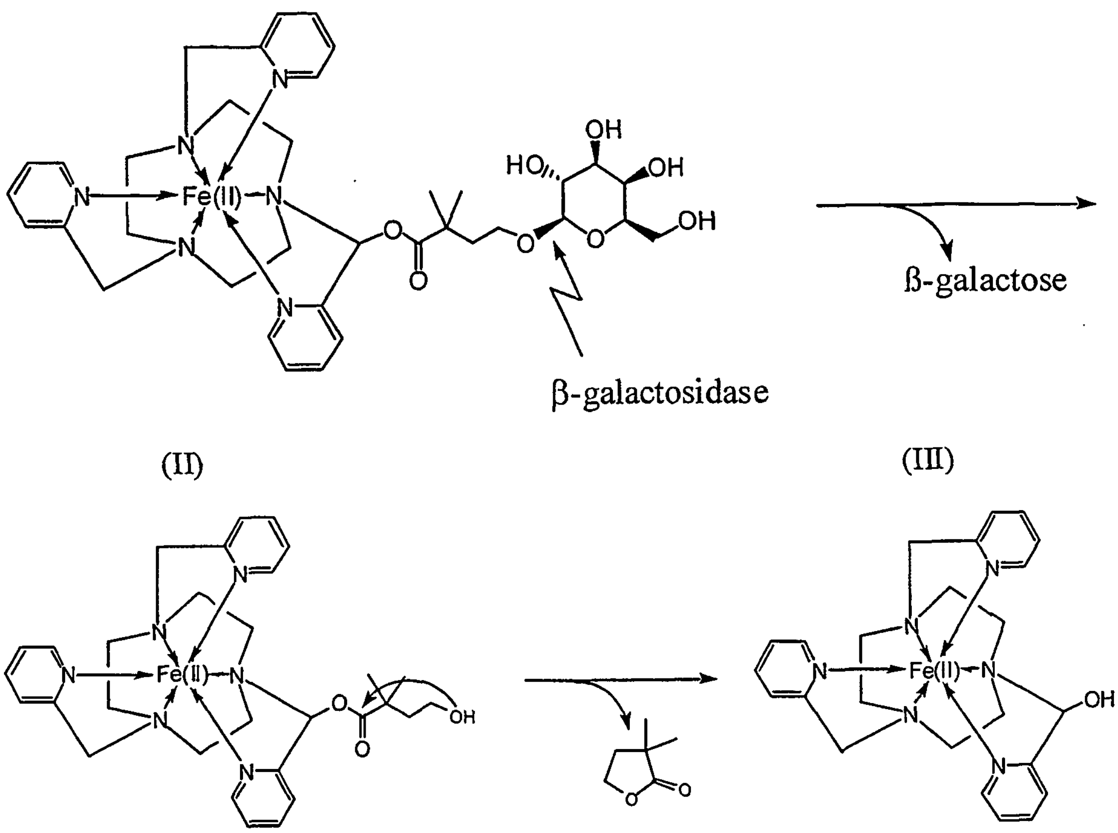


(II)

(III)

(IV) (V)

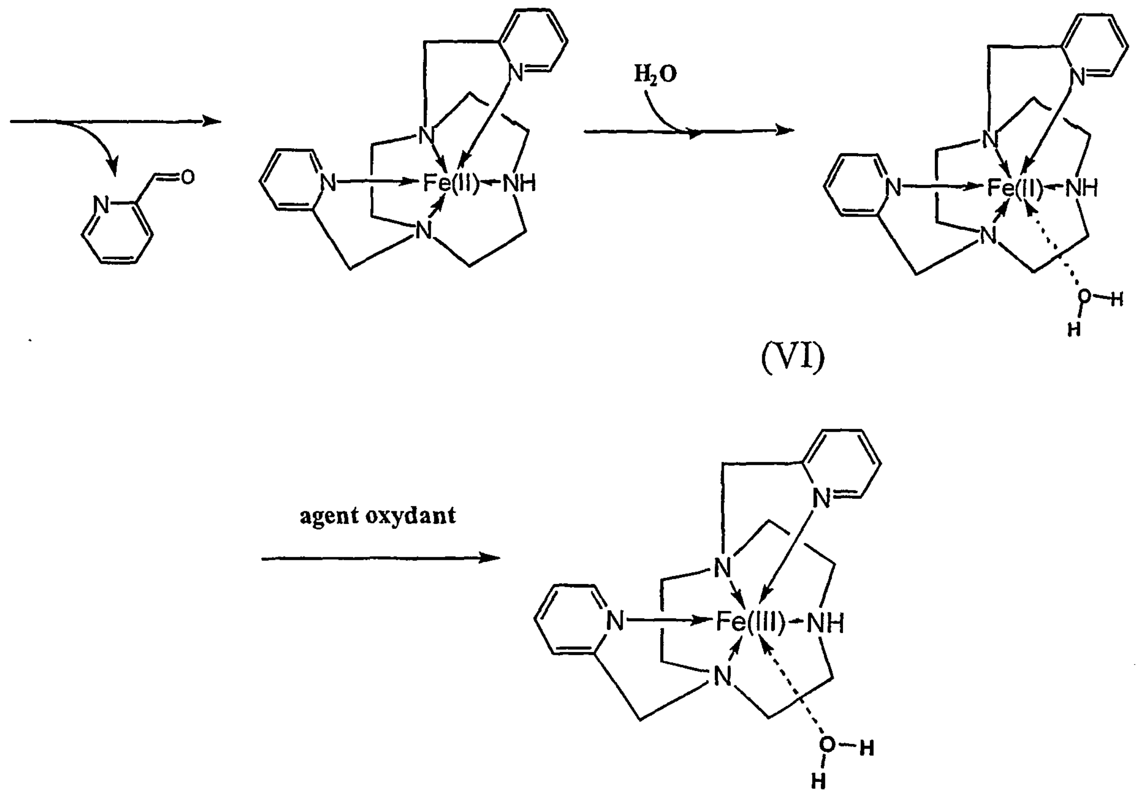


(VI)

Cette technique permet de détecter, outre la β-galactosidase, la présence de substances cibles très variées. On rapporte ci-après des exemples de groupes E-$R^6$ utiles pour détecter la présence de certaines substances cibles particulières, ou tout autre enzyme faisant partie des classes auxquelles les substances cibles suivantes appartiennent, notamment les glycosidases, les aminopeptidases, les lipases, et les phosphatases :

| **E-$R^6$** | **Substance cible** |
|---|---|
| 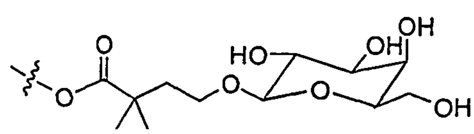 | β-galactosidase (EC 3.2.1.23) |
| 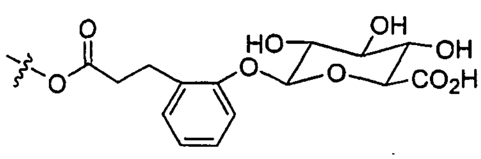 | β-glucuronidase (EC 3.2.1.31) |
| 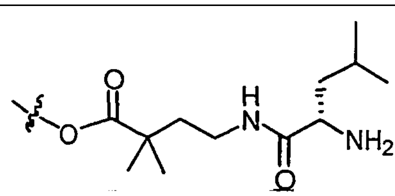 | Leucyl aminopeptidase (EC 3.4.11.1) |
| 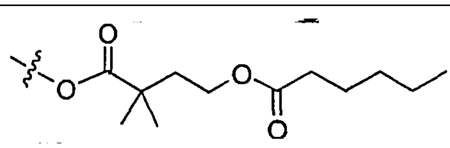 | lipases (EC 3.1.1.3) |

(suite)

| E-$R^6$ | Substance cible |
|---|---|
| 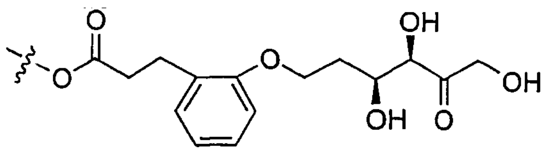 | transaldolases (EC 2.2.1.2) (voir par exemple E. Gonzalez-Garcia et al. Chem. Eur. J. 2003, 9, 893-899) |
| 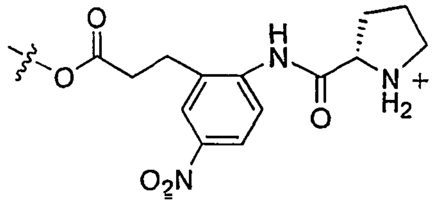 | prolyl aminopeptidase (EC 3.4.11.5) |
| 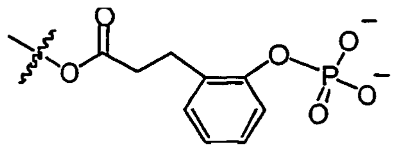 | alcaline phosphatase (EC 3.1.3.1) |

Le code EC est celui utilisé par « International Union of Biochemistry and Molecular Biology IUBMB ».

On peut trouver les significations des exemples ci-dessus dans des bases de données telles que celles répertoriées dans http://www.brenda.uni-koeln.de ou http://au.expasy.org/enzyme/.

De même, l'action d'un complexe de type (Ia-2) dans lequel E est un groupe espaceur (-$C_6X_4$-Y-) tel que défini plus haut, et $R^6$ est un groupe β-galactosyle est illustrée par le schéma réactionnel 2 ci-dessous.

Lorsque le complexe (Ia-2) est mis en présence de β-galactosidase, il se produit une coupure du groupe β-galactosyle avec formation de β-galactose, puis une coupure spontanée entre l'atome de carbone qui portait le substituant $R^5$ et l'atome d'azote du macrocycle, avec formation de quinone-méthide. Une molécule d'eau peut alors se fixer sur le site de coordination libéré, puis l'action d'un agent oxydant permet de transformer le complexe de Fer (II) en complexe de Fer(III).

Schéma 2

(Ia)

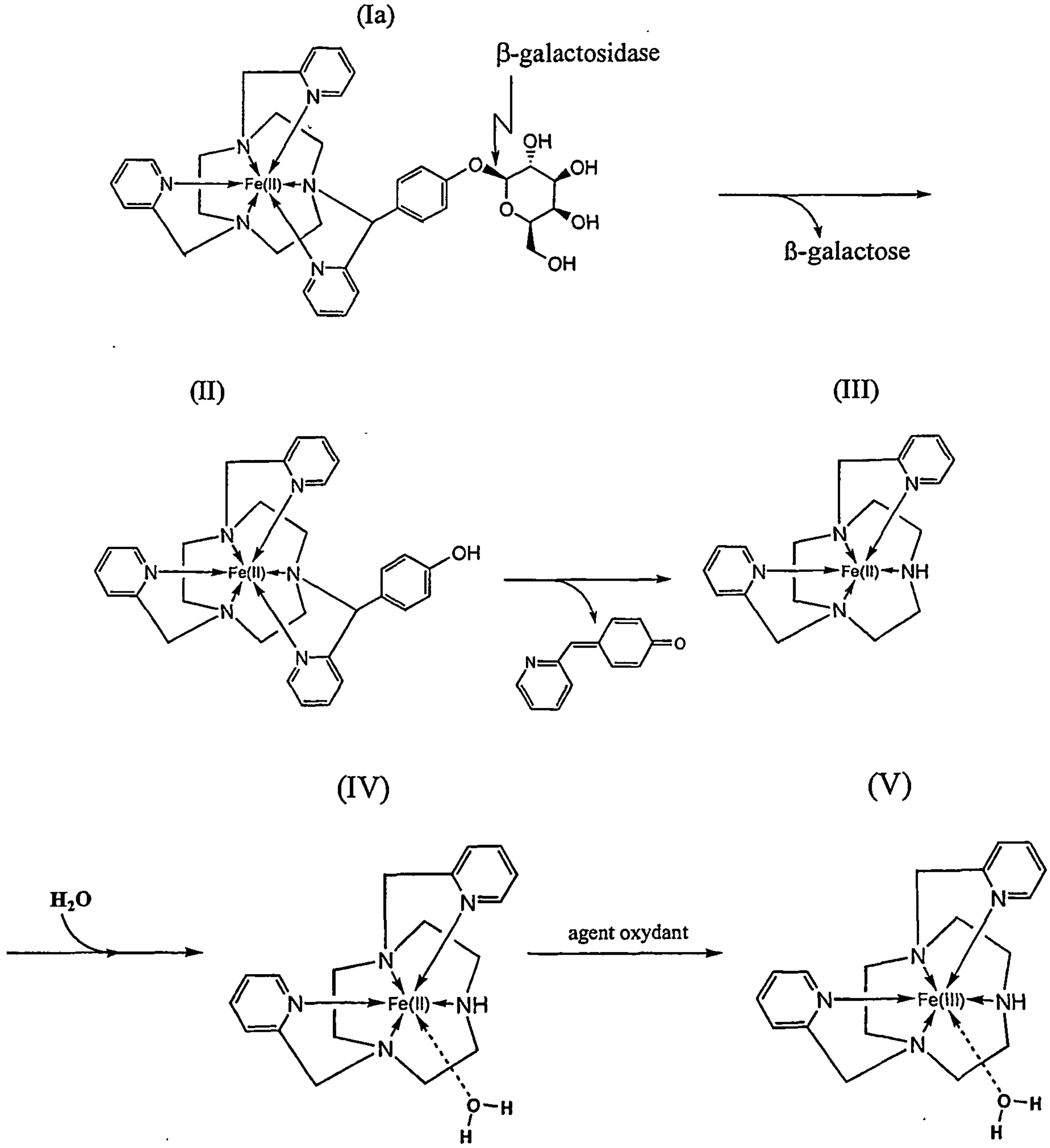


(II)

(III)

(IV)

(V)

Il est également possible dans ce cas de détecter, outre la β-galactosidase, la présence de substances cibles très variées. On rapporte ci-après des exemples de groupes $R^6$ utiles pour détecter la présence de certaines substances cibles particulières, ou tout autre enzyme faisant partie des classes auxquelles les substances cibles suivantes appartiennent, notamment les glycosidases, les aminopeptidases, les lipases et les phosphatases :

| $R^6$ | Substance cible |
|---|---|
| 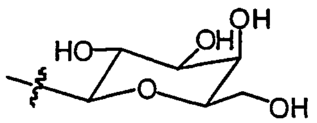 | β-galactosidase (EC 3.2.1.23) |
| 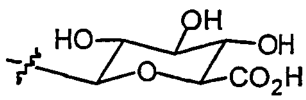 | β-glucuronidase (EC 3.2.1.31) |
| 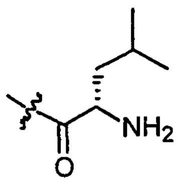 | Leucyl aminopeptidase (EC 3.4.11.1) |
| 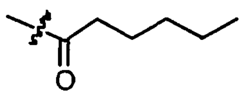 | lipases (EC 3.1.1.3) |
| 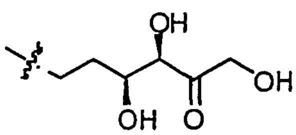 | transaldolases (EC 2.2.1.2) (voir par exemple E. Gonzalez-Garcia et al. Chem. Eur. J. 2003, 9, 893-899) |
| 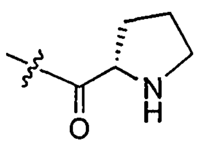 | prolyl aminopeptidase (EC 3.4.11.5) |
| 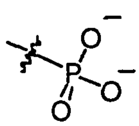 | alcaline phosphatase (EC 3.1.3.1) |

L'action d'un complexe (Ib) qui comprend un ion Fe et un ligand du type hexadentate portant un substituant $R^i$ capable de subir une modification électronique est illustrée par le schéma réactionnel ci-dessous (schéma 3), pour un complexe dans lequel l'un des substituants $R^i$ est un groupe $-CH(OH)CH(COOH)NH_2$ et les autres substituants $R^i$ sont des groupes $-NO_2$. Un tel substituant se réorganise sous l'action de la L-thréonine aldolase en libérant de la glycine, ce qui provoque un changement d'état de spin. Il permet par conséquent de détecter la L-thréonine aldolase.

**Schéma 3**

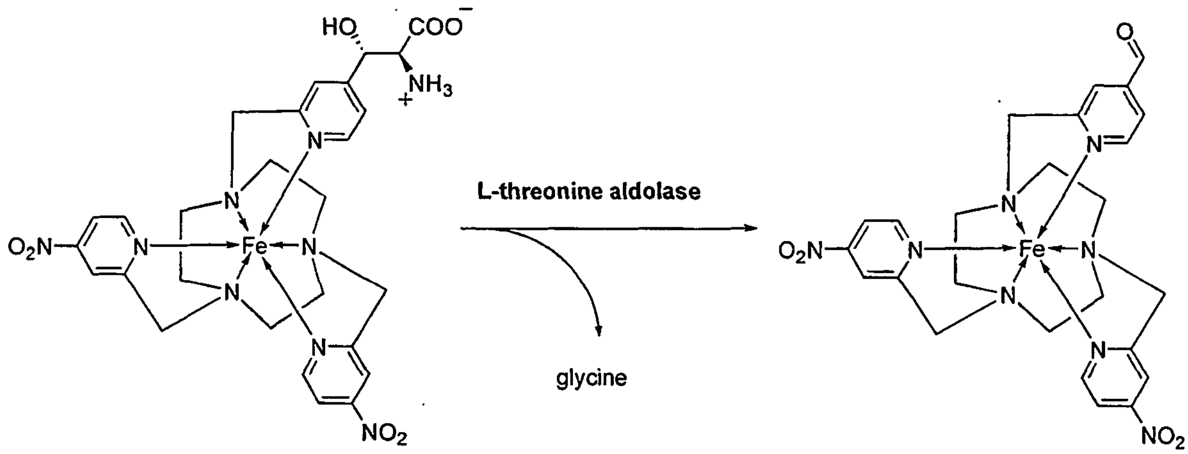


De même, l'action d'un complexe de type (II), dans lequel E est un groupe espaceur ($-C_6X_4-Y-$) tel que défini plus haut, W n'est pas un groupe particulier (m=0), et $R^6$ est un groupe β-galactosyle, est illustrée par le schéma réactionnel 4 ci-dessous.

Lorsque le complexe (II) est mis en présence de β-galactosidase, il se produit une coupure du groupe β-galactosyle avec formation de β-galactose, puis un réarrangement du groupement espaceur de sorte à former une quinone-méthide, qui entraîne l'élimination du groupe benzotriazole. Une molécule d'eau peut alors se fixer sur le site de coordination libéré, puis l'action d'un agent oxydant permet de transformer le complexe de Fer (II) en complexe de Fer(III).

**Schéma 4**

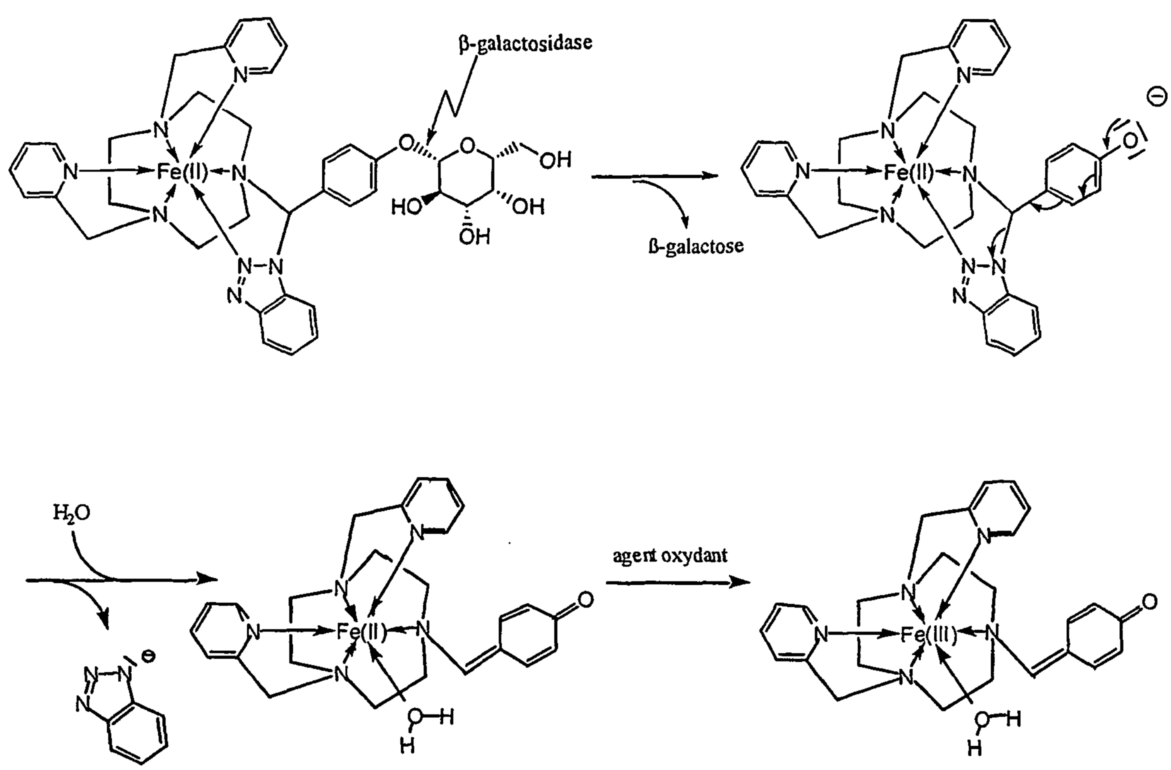


L'invention a également pour objet une composition pharmaceutique qui comprend un agent de contraste

selon l'invention.

Elle concerne aussi l'utilisation d'un agent de contraste selon l'invention pour la préparation d'une composition pharmaceutique destinée au diagnostic par imagerie par résonance magnétique, ainsi que la méthode d'imagerie par résonance magnétique d'une cellule ou d'un tissu auquel un agent de contraste selon l'invention a été administré.

En outre, l'invention a pour objet une composition pharmaceutique destinée à la détermination de la distribution tissulaire de β-galactosidase ou de β-glucuronidase, dans laquelle l'agent de contraste répond à la formule (Ia), dans laquelle $R^5$ représente un groupe -E-$R^6$, ou un groupe ($C_6X_4$-Y)-$R^6$, $R^6$ représentant respectivement un groupe β-galactosyle ou un groupe β-glucuronyle, et les groupes E, ($C_6X_4$-Y), $R^i$ et $R^4$ sont tels que définis précédemment.

L'invention a également pour objet une composition pharmaceutique destinée à la détermination de la distribution tissulaire d'aminopeptidases, de lipases, de transaldolases, et de phosphatases, dans laquelle l'agent de contraste répond à la formule (Ia), dans laquelle $R^5$ représente un groupe -E-$R^6$, ou un groupe ($C_6X_4$-Y)-$R^6$, $R^6$ représentant un groupe *L*-leucyle, un groupe -$COC_5H_{11}$, un groupe de type α,β-dihydroxyacétone

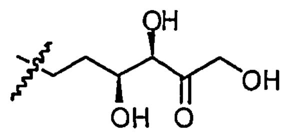

,

un groupe prolyle, ou un groupe phosphoryle, et les groupes E, ($C_6X_4$-Y), $R^i$ et $R^4$ sont tels que définis précédemment.

De même, l'invention a pour objet une composition pharmaceutique destinée à la détermination de la distribution tissulaire de *L*-thréonine aldolase, dans laquelle l'agent de contraste répond à la formule (Ib).

<u>Préparation d'un complexe de type (Ia) dans lequel $R^5$ est un groupe -O-CO-C($CH_3$)$_2$-($CH_2$)$_2$-O$R^6$</u>

La préparation d'un complexe (Ia-1) dans lequel $R^6$ est un groupe β-galactosyle et les autres substituants $R^i$ et $R^4$ sont des atomes d'hydrogène, peut être effectuée comme illustré par le schéma 5.

**Schéma 5**

1 2

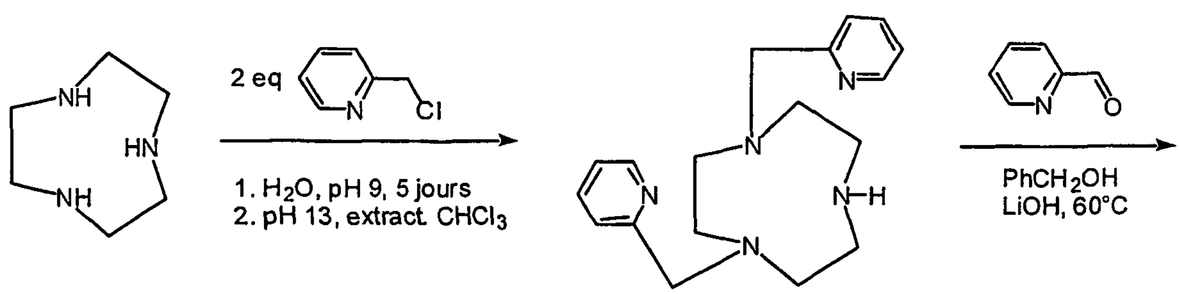

3 4

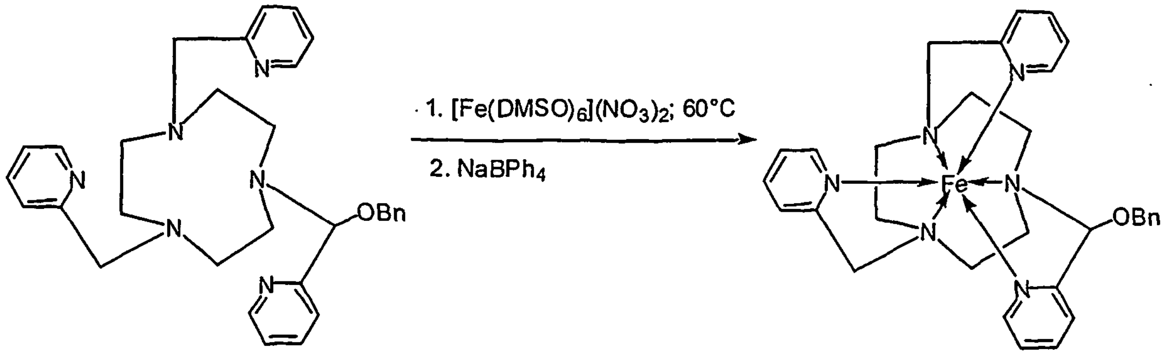


5 6

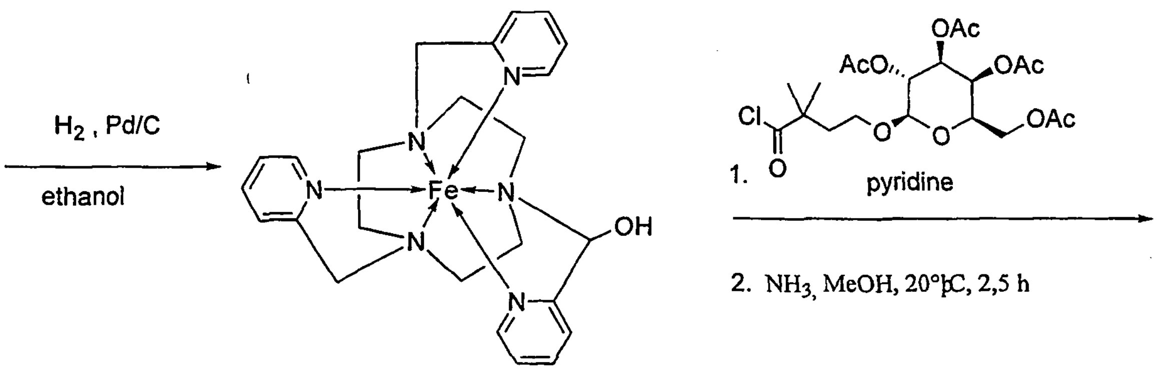


Ia

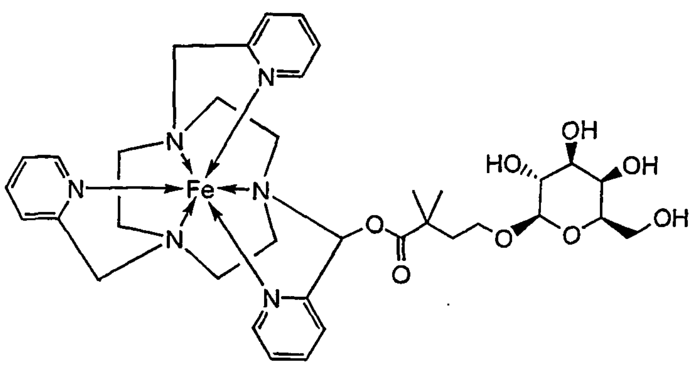

Le composé (2) est préparé en faisant réagir le 1,4,7-triazacyclononane 1 disponible dans le commerce avec deux équivalents de chlorométhylpyridine en milieu aqueux à pH 9 pendant 3 jours ; à ce point le pH a baissé à 7. On ajuste le pH encore à 9 et on agite 3 jours de plus avant d'extraire le composé (2), selon le mode opératoire décrit par Spiccia, et al., Inorg. Chem. 1994, 33, 4663.

Le composé (2) est ensuite transformé en N/O-acetal benzylé (3) par réaction avec la pyridine-2-carbaldéhyde dans l'alcool benzylique, en présence de LiOH à 60˚C, selon un procédé publié pour un produit analogue par Ugalde-Saldivar et al. (J. Chem. Soc. Dalton Trans. 2001, 3099-3107).

Ensuite, on transforme le composé (3) en complexe de fer (4) par réaction avec $[Fe(DMSO)_6](NO_3)_2$ à 60˚C dans du méthanol pendant 30 minutes, puis on ajoute du $NaBPh_4$ avant de refroidir à 5˚C pour précipiter le complexe et l'isoler.

Après élimination du groupe benzyle par l'hydrogène en présence de palladium sur charbon dans de l'éthanol, on obtient le composé (5) que l'on fait réagir avec le chlorure d'acyle (6) (voir sa synthèse ci-dessous) dans la pyridine

et l'on obtient un complexe dans lequel le groupe galactosyle est sous forme protégée. La déprotection est effectuée par action de l'ammoniaque aqueux dans le méthanol à 20°C pendant 2,5 heures et l'on obtient le complexe de formule (Ia) souhaité.

La présente invention concerne également le composé (4), utile en tant qu'intermédiaire de synthèse.

Pour synthétiser le chlorure d'acyle (6) on peut procéder selon le schéma 6 suivant :

**Schéma 6**

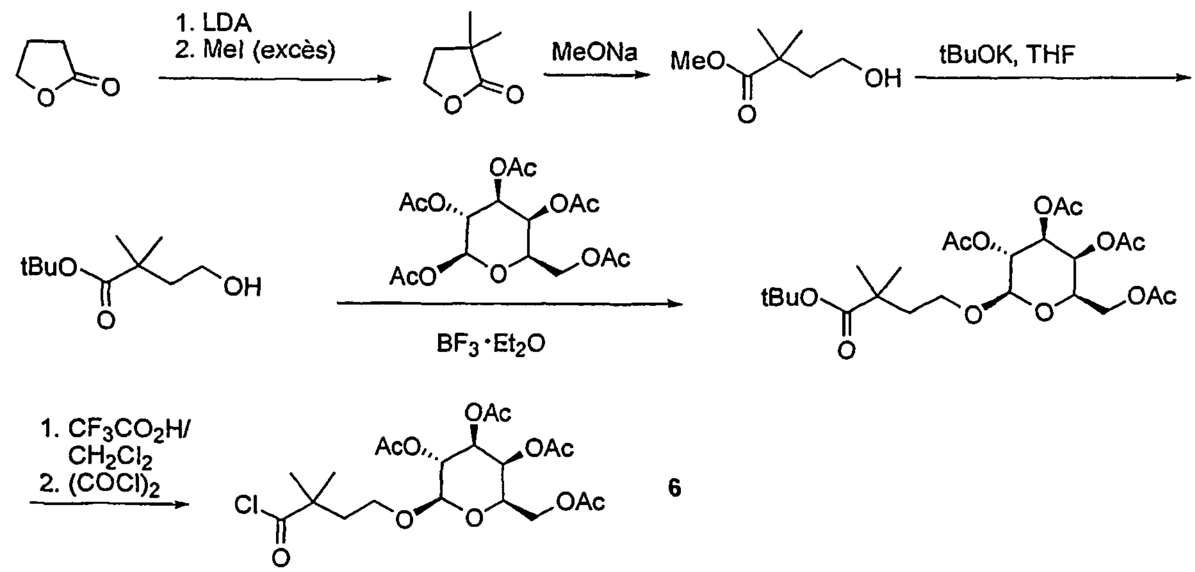


6

Pour obtenir un complexe de formule (Ia), dans laquelle l'espaceur est de type (E2), le précurseur analogue du chlorure d'acyle (6) peut être synthétisé selon le schéma 7 suivant :

**Schéma 7**

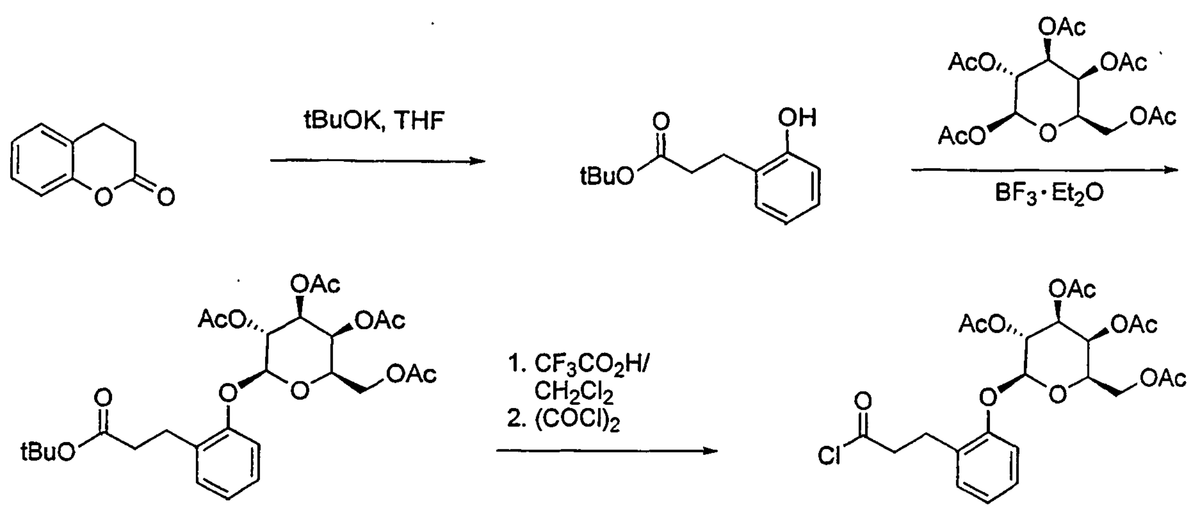


Pour obtenir des complexes de formule (Ia), dans laquelle les substituants $R^i$ ne sont pas des atomes d'hydrogène, on peut procéder de la même façon en utilisant de la chlorométhylpyridine substituée avec les groupes $R^i$ souhaités. Par exemple on pourra se reporter au schéma 8 pour la préparation de la chlorométhylpyridine para-nitrosubstituée comme produit de départ dans la première étape du schéma 5.

Pour obtenir des complexes de formule (Ia), dans laquelle E-$R^6$ a une définition plus générale -O-CO-$(CR'_2)_n$-Y-$R^6$ avec R', n, Y et $R^6$ tels que précédemment définis, on procède comme dans le schéma 5 par couplage d'un composé (4) préalablement hydrogéné, avec un chlorure d'acyle de formule Cl-CO-$(CR'_2)_n$-Y-$R^6$ en présence d'une base. On obtient alors un complexe de formule (Ia) éventuellement protégé, que l'on peut ensuite déprotéger selon les techniques classiques de l'homme de l'art.

<u>Préparation des complexes de type (Ia) dans lequel $R^5$ est un groupe $C_6H_4$-$OR^6$</u>

La préparation d'un complexe (Ia-2) dans lequel $R^6$ est un groupe β-galactosyle et les autres substituants $R^i$ et $R^4$ sont des atomes d'hydrogène, peut être effectuée comme illustré par le schéma 8.

**Schéma 8**

1'

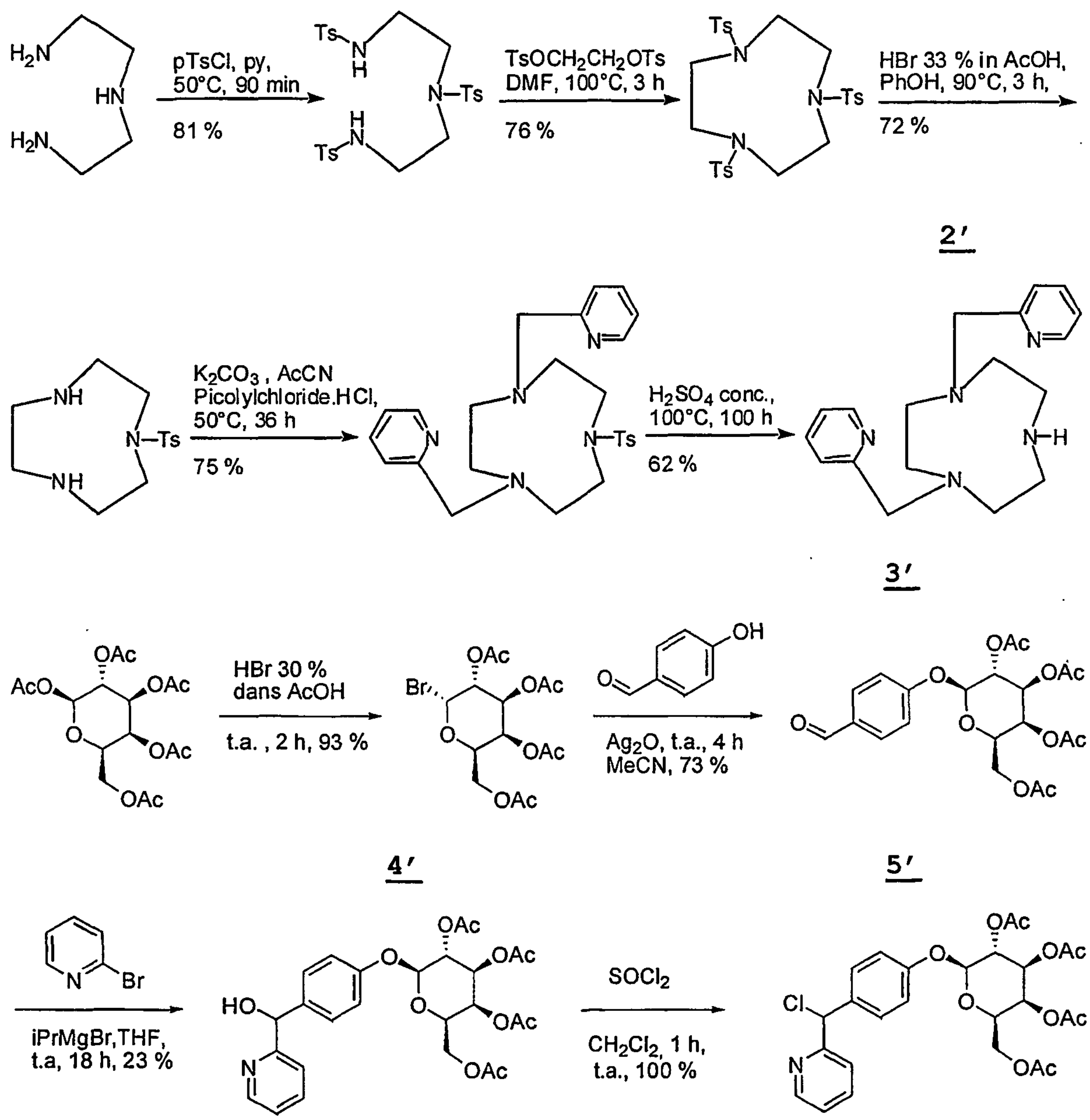

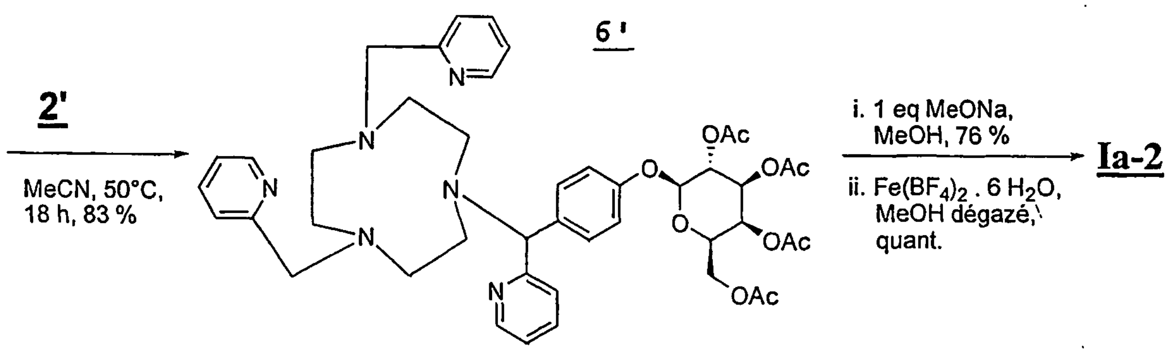


Le composé (2') est préparé en faisant réagir la diéthylènetriamine (disponible dans le commerce) avec le chlorure de tosyle. Le composé résultant est traité dans une première étape avec du MeONa et ensuite dans une étape indépendante par le bis-tosylate d'éthylèneglycol pour fournir le produit (1'). Deux atomes d'azote de (1') peuvent être sélectivement déprotégés par l'action de l'acide bromhydrique dans l'acide acétique glacial. Un traitement par l'hydrochlorure du chlorure de picolyle introduit les deux bras picolyle sur le macrocycle azoté, et le dernier groupe tosyle est éliminé par un traitement dans l'acide sulfurique concentré à 100°C pour fournir le composé (2').

Le composé (3') est obtenu en faisant réagir en présence d'oxyde d'argent le 4-hydroxybenzaldéhyde (disponible dans le commerce) avec la 1-bromo-tétra-O-acetyl-β-D-galactopyranose, celle-ci étant obtenue par un traitement de la penta-*O*-acétyl-β-D-galactopyranose (disponible dans le commerce) par l'acide bromhydrique dans l'acide acétique glacial.

Ensuite, le composé (3') est traité par un mélange d'un équivalent de 2-bromopyridine et d'un équivalent du bromomagnésien d'isopropyle pour obtenir l'alcool (4'). Celui-ci peut être transformé en chlorure (5') par réaction avec du chlorure de thionyle. Le composé (5') sert à alkyler le composé (2') avec assistance du carbonate de potassium pour obtenir le composé (6'). Celui-ci est traité avec du MeONa dans le méthanol pour éliminer les quatre groupements protecteurs acétyle sur l'unité galactosyle. La synthèse multi-étape du complexe Ia-2 se termine avec le traitement par $Fe(BF_4)_2$ dans le méthanol pour complexer le ligand avec un atome de Fer(II).

La préparation des différents composés formés au cours de cette synthèse est illustrée par les exemples 1 à 14.

Pour obtenir des complexes de formule (Ia-2), dans laquelle les substituants $R^i$ ne sont pas des atomes d'hydrogène, on peut procéder de la même façon en utilisant de la chlorométhylpyridine substituée avec les groupes $R^i$ et/ou $R^4$ souhaités. Par exemple on pourra se reporter au schéma 9 pour la préparation de la chlorométhylpyridine para-nitrosubstituée comme produit de départ dans la première étape du schéma 8.

Pour obtenir des complexes de formule (Ia-2), dans laquelle E-$R^6$ a une définition plus générale -$C_6X_4$-Y-$R^6$ avec X, Y et $R^6$ tels que précédemment définis, on procède comme dans le schéma 8 en remplaçant le réactif penta-*O*-acetyl-β-D-galactopyranose par un chlorure d'acyle pour les unités $R^6$ répondant à une activité estérase/lipase, et par un acide aminé protégé et activé pour une réponse à l'activité de protéases. Pour l'unité $R^6$ représentant une α,β-dihydroxycétone, la stratégie décrite dans la publication de E. Gonzalez-Garcia et al. Chem. Eur. J. 2003, 9, 893-899 peut être appliquée.

Pour obtenir des complexes de formule (Ia-2), dans laquelle $R^4$ représente un groupement méthyle, il faut synthétiser un dérivé correspondant du chlorure de picolyle introduit dans la première étape du schéma 8.

<u>Préparation des complexes de type (Ib) dans lequel $R^5$ est un atome d'hydrogène et l'un des substituants $R^i$ est un groupe -CH(OH)CH(COOH)$NH_2$</u>

Un complexe (Ib) dans lequel $R^5$ est un atome d'hydrogène, l'un des substituants $R^i$ est un groupe -CH(OH) CH(COOH)$NH_2$ et les substituants $R^i$ restants sont des groupes -$NO_2$, peut être obtenu à partir du cyclène (1) précité, et des réactifs préparés au préalable.

Un réactif (7) peut être préparé selon le schéma réactionnel suivant (schéma 9). La dernière étape est effectuée sous reflux de dichlorométhane.

Schéma 9

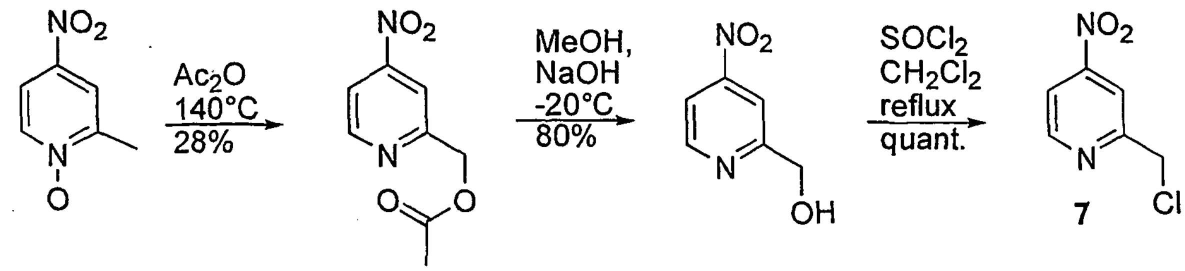


7

Un réactif (8) peut être préparé selon le schéma réactionnel suivant (schéma 10). La quatrième étape est effectuée en chauffant le produit de départ avec le dioxyde de sélénium dans le dioxane pendant 4 jours (Bishop et al. Tetrahedron 2000, 4629-4638).

Schéma 10

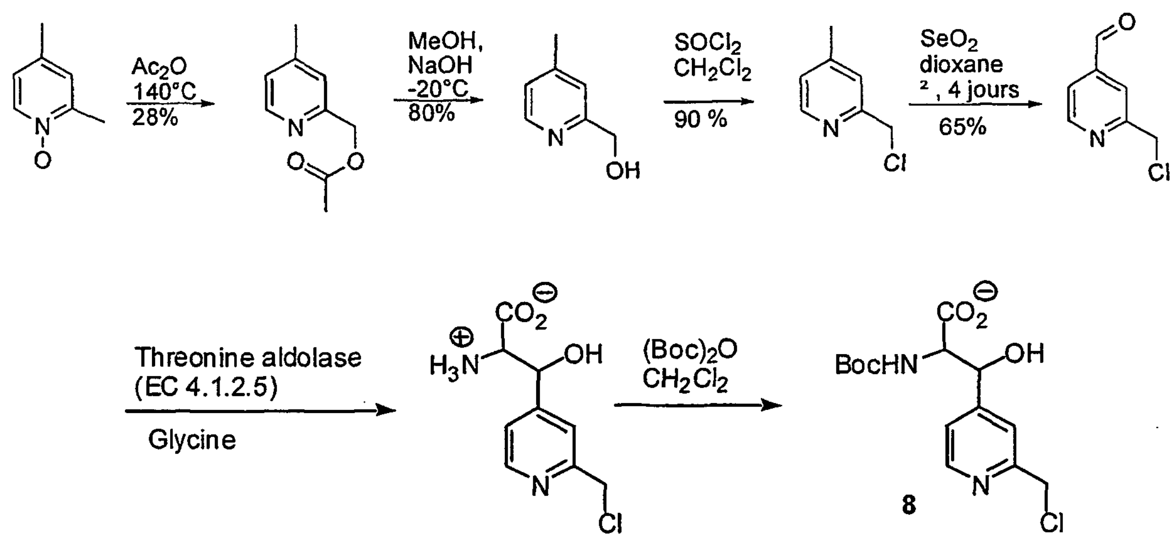


8

La préparation du complexe peut être effectuée selon le mode opératoire suivant, illustrée par le schéma 11 :

**Schéma 11**

1 9

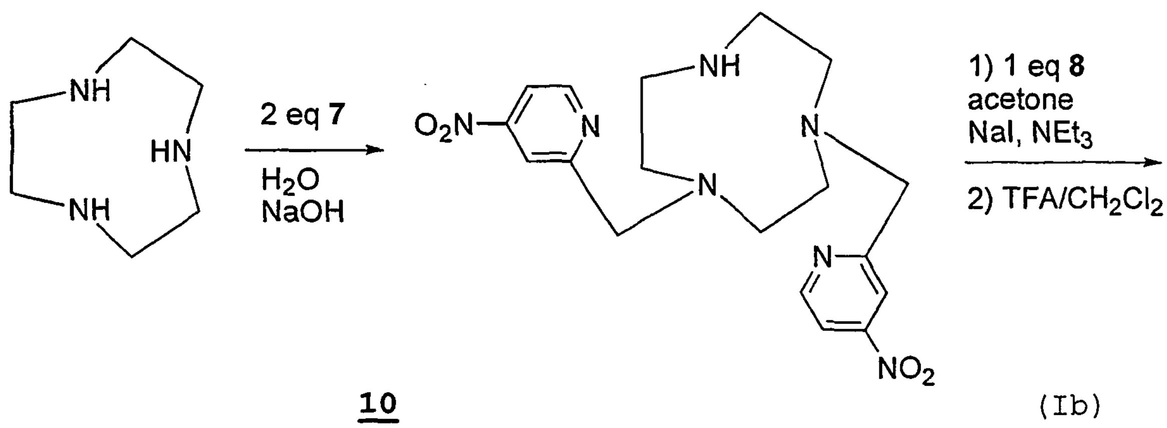


10 (Ib)

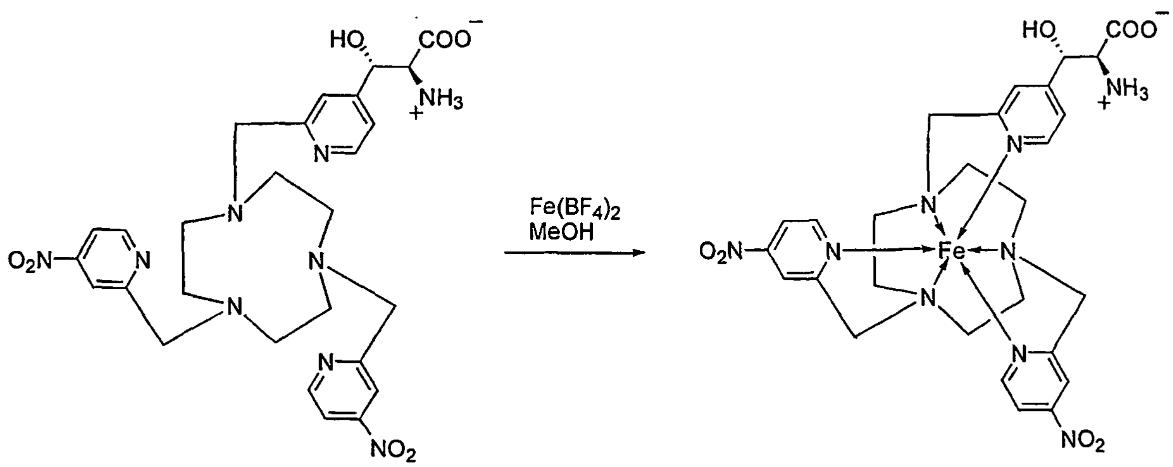


On procède selon le même mode opératoire que celui décrit dans le schéma 5 pour obtenir le composé de formule (9). Ensuite, on fait réagir le composé (9) avec un équivalent de composé (8) en présence de NaI et $NEt_3$ dans l'acétone. La déprotection du groupement amine primaire par l'acide trifluoroacétique dans le dichlorométhane conduit au composé (10). Le composé (10) est transformé en complexe de fer par action de $Fe(BF_4)_2$ dans le méthanol, et l'on obtient le complexe de formule (Ib).

La présente invention concerne également le composé (9), utile en tant qu'intérmédiaire de synthèse.

Préparation des complexes de type II

La préparation d'un complexe de type II dans lequel $R^5$ est un groupement $-C_6H_4$-O-β-galactosyle, les deux atomes d'azote et le groupement Z constituent ensemble une unité benzotriazole, W ne représente rien (m=0), et $R^1$, $R^2$ et $R^4$ représentent des atomes d'hydrogène, peut être effectuée comme illustré par le schéma 12.

**Schéma 12**

1"

2'

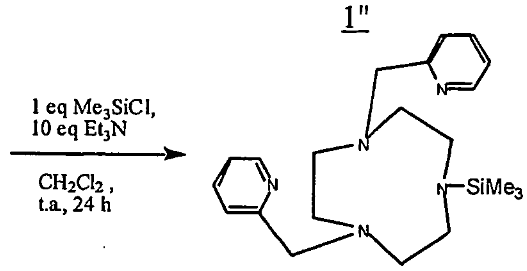


2"

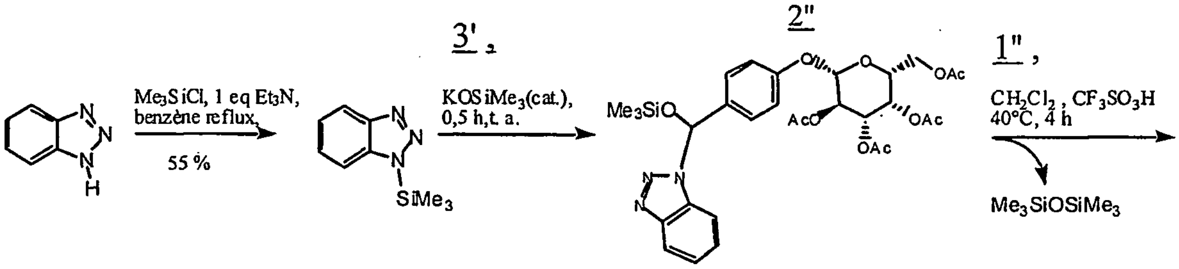


3"

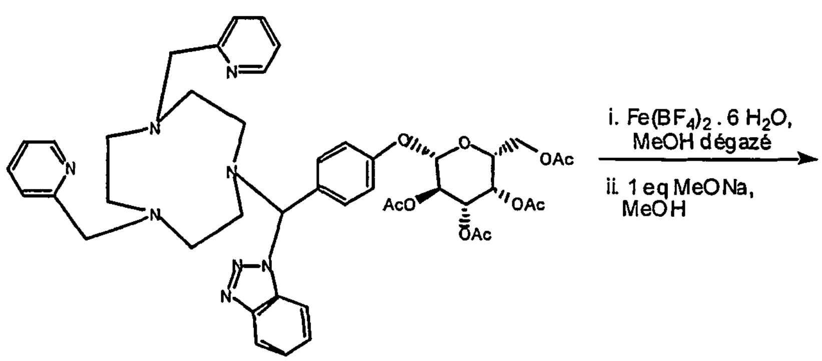


**II**

Le composé (1") est obtenu en faisant réagir le produit (2'), décrit dans le schéma 8, avec le chlorotriméthylsilane en présence d'un large excès de triéthylamine, selon un mode opératoire publié pour une autre amine secondaire (P.B. Rasmussen et al., Bull. Soc. Chem. Fr. 1985, II, 62-64). Après filtration, évaporation de tout produit volatile, redissolution dans le dichlorométhane et filtration des produits solides résiduels, le composé (1") est directement introduit dans la réaction avec le produit (2"). Celui-ci est tout d'abord synthétisé en fabriquant le 1-triméthylesilylbenzotriazole selon un mode opératoire décrit dans la littérature (Inorg. Chem. 1989, 28, 4022-4026), et en faisant réagir ce dernier avec le composé (3') décrit dans le schéma 8 selon un mode opératoire publié (A. Bourry et al., J. Heterocylic Chem. 2002, 39, 109-118).

Les composés (1") et (2") sont transformés en composé (3") selon un mode opératoire décrit dans la même publication (A. Bourry et al., J. Heterocylic Chem. 2002, 39, 109-118). La dernière étape consiste à traiter le composé (3") par le $Fe(BF_4)_2$ dans le méthanol, puis par du MeONa dans le méthanol pour éliminer les 4 groupements protecteurs acétyle sur l'unité galactosyle. Le complexe II résultant est purifié par évaporation du mélange réactionnel, redissolution dans l'eau, et passage à travers une colonne commerciale remplie de silice phase inverse (C18).

L'invention est décrite plus en détail par les exemples suivants, auxquels elle n'est cependant pas limitée.

Les exemples 1 à 14 décrivent la préparation des différents composés intervenant dans la synthèse des complexes de type Ia-2, selon le schéma 8.

**Exemple 1**

**Préparation du bis(p-toluènesulphonyl) éthylène glycol**

On dissout 19,8 g (0,3 mol) d'éthylène glycol dans 150 ml de pyridine distillée puis on refroidit la solution avec un bain de glace. On ajoute lentement, sous agitation mécanique, du chlorure de l'acide p-toluènesulfonique (123 g; 0,65 mol). La température doit rester inférieure à 20˚C pendant l'addition. Lorsqu'environ 90g ont été ajoutés, l'agitation devient difficile et une dilution à l'aide de 200 ml de pyridine est effectuée. Le mélange réactionnel est laissé sous agitation pendant 2 h 30. On ajoute une solution aqueuse de HCl (12 N HCl -170 ml - dans la glace - 500ml -). On filtre le solide formé au court de cet addition. On dissout ce solide dans du méthanol bouillant (250 ml), puis on filtre le nouveau précipité formé après retour à température ambiante. Le solide blanc obtenu est enfin séché sous vide et l'on obtient le produit pur (92,4 g ; rendement 83%).
Caractéristiques : RMN $^1H$ (200 MHz, $CDCl_3$) δ 7,71 (d, 2H) ; 7,31 (d, 2H) ; 4,16 (s, 2H) ; 2,43 (s, 3H).

**Exemple 2**

**Préparation de la tris-(p-toluènesulfonyl) diéthylènetriamine**

On dissout 115 g (0,6 mol) du chlorure de l'acide p-toluènesulfonique dans 250 ml de pyridine à température ambiante. On ajoute une solution de diéthylène triamine (21,5 ml, 0,2 mol) dans la pyridine (30 ml) au goutte-à-goutte. Le mélange réactionnel est agité à 50˚C pendant 90 minutes, puis transféré à chaud dans un Erlenmeyer. Après retour à température ambiante, on ajoute 200 ml d'eau. On laisse le mélange sous agitation pendant la nuit à température ambiante puis on le place dans un bain de glace pendant 2 heures. On filtre le solide orange qui se forme alors, on le lave avec de l'éthanol à 95% froid (0˚C). Après séchage sous vide à 40˚C (rotavapor), le produit désiré est obtenu sous forme d'un solide jaune clair (92 g ; rendement 81%).

**Exemple 3**

**Préparation du sel disodique de la tris-(p-toluènesulfonyl)diéthylènetriamine**

On prépare une solution d'éthanolate de sodium par addition lente de morceaux de sodium (7,5 g ; 0,32 mol) dans l'éthanol absolu (220 ml). On dissout la tris-(p-toluènesulfonyl)diéthylènetriamine (92,0 g ; 0,16 mol) dans de l'éthanol absolu (220 ml) et on porte le mélange à reflux. Le chauffage est arrêté et on ajoute en une fois la solution d'éthanolate de sodium. Les impuretés insolubles sont éliminées par décantation. On laisse la solution cristalliser pendant la nuit. Les cristaux obtenus sont filtrés sous argon, lavés avec de l'éthanol absolu, puis séchés sous vide à 100˚C. Le sel disodique est obtenu sous forme de cristaux blancs (98,0 g ; rendement 98%).

**Exemple 4**

**Préparation du 1,4,7-tris(p-toluènesulfonyl)-1,4,7-triazacyclononane (composé 1')**

On ajoute goutte-à-goutte, sur une durée de 3 heures, une solution de bis (p-toluènesulphonyl) éthylène glycol (59,5 g ; 0,16 mol) dans le DMF (300 ml) à une solution du sel disodique de la tris-(p-toluènesulfonyl) diéthylènetriamine (98g ; 0,16 mol) dans le DMF (950 ml) à 100˚C. Le chauffage est arrêté et on ajoute de l'eau distillée (575 ml). Un précipité se forme immédiatement. Après filtration et séchage sous vide, le composé macrocyclique est obtenu sous forme d'un solide blanc (71,8g; rendement 76%).
Caractéristiques :
RMN $^1H$ (200 MHz, $CDCl_3$) δ 7,67 (d, 6H) ; 7,29 (d, 6H) ; 3,39 (s, 12H) ; 2,41 (s, 9H).

**Exemple 5**

**Préparation du 1-(p-toluènesulfonyl)-1,4,7-triazacyclononane**

On dissout du 1,4,7-tris(p-toluènesulfonyl)-1,4,7-triazacyclononane (17,56 g ; 29,7 mmol) et du phénol (21 g, 223,0 mmol) dans 240 ml d'une solution de HBr (33%) dans l'acide acétique glacial. La solution est chauffée à 90˚C pendant 30 h. Un solide apparaît après 3 h. Après retour à température ambiante, on filtre le mélange et on lave le solide avec de l'éther diéthylique (2x80 ml). On dissout le sel hydrobromique dans une solution de soude 1 M jusqu'à ce que le pH soit de l'ordre de 12 unités. On extrait la solution aqueuse à l'aide de chloroforme (8x50 ml). On sèche les

phases organiques réunies sur $Na_2SO_4$ et on évapore le solvant sous vide. On obtient finalement un solide blanc (7,36 g, rendement 88%).

**Exemple 6**

**Préparation du 1-(p-toluènesulfonyl)-4,7-(pyridinylméthyl)-1,4,7-triazacyclononane**

A une suspension de 1-(p-toluènesulfonyl)-1,4,7-triazacyclononane (3,37 g ; 11,91 mmol) et de $K_2CO_3$ (12 g ; 86,83 mmol) dans 60 ml d'acétonitrile distillé, on ajoute de l'hydrochlorate de chloropicoline (4,30 g ; 26,20 mmol). On laisse le mélange sous agitation pendant 36 h à 50˚C sous argon. Le sel obtenu est filtré puis lavé par de l'acétonitrile 2x10 ml. On évapore le solvant sous vide puis on purifie le produit par chromatographie sur gel de silice (gradient de $CH_2Cl_2$/MeOH de 15/1 à 5/1) pour obtenir le produit pur sous forme d'une huile jaune (2,98 g ; rendement 75%).
Caractéristiques :
RMN $^1H$ (200 MHz, $CDCl_3$) δ 8,49 (d, 2H) ; 7,63 (m, 4H) ; 7.47 (d, 2H) ; 7,26 (t, 2H) ; 7,13 (t, 2H) ; 3,90 (s, 4H); 3,19 (dd, 8H) ; 2,80 (s, 4H) ; 2,40 (s, 3H) ;
RMN $^{13}C$ (50 MHz, $CDCl_3$) δ 159,83 ; 148,81 ; 142,82 ; 136,23 ; 135,81 ; 129,44 ; 126,93 ; 123,06 ; 121,77 ; 63,65 ; 55,65 ; 50,73 ; 21,29.

**Exemple 7**

**Préparation du 1,4-dipyridinylméthyl-1,4,7-triazacyclononane (composé 2')**

On dissout le 1-(p-toluènesulfonyl)-4,7-(pyridinylméthyl)-1,4,7-triazacyclononane (502 mg ; 1,08 mmol) dans 5 ml de $H_2SO_4$ concentré sous argon. On laisse le mélange sous agitation pendant 32 h à 100˚C. Après retour à la température ambiante, on refroidit la solution à l'aide d'un bain de glace puis on ajoute lentement une solution de NaOH à 50% jusqu'à ce que le pH soit voisin de 12. On extrait la phase aqueuse par 3x150 ml de chloroforme. La phase organique est séchée sur $Na_2SO_4$ et évaporée sous vide pour obtenir une huile jaune (255 mg ; rendement 76%).
Caractéristiques :
RMN $^1H$ (200 MHz, $CDCl_3$) δ 8,57 (d, 2H) ; 7,53 (t, 2H) ; 7,15 (dd, 4H) ; 3,85 (s, 4H) ; 3,15 (t, 4H) ; 2,95 (t, 4H) ; 2,65 (s, 4H) ;
RMN $^{13}C$ (50 MHz, $CDCl_3$) δ 157,2 ; 149,29 ; 136,72 ; 122,76 ; 122,45 ; 60,81 ; 52,65 ; 49,39 ; 45,20.

**Exemple 8**

**préparation de tétraacétyl-α-bromogalactose**

On dissout le β-pentaacétyl-galactose (10 g ; 25,5 mmol) dans 25 ml d'une solution de HBr (33%) dans l'acide acétique et 5 ml d'anhydride acétique. On laisse la solution sous agitation pendant 2 heures à température ambiante. On ajoute 200 ml de $CH_2Cl_2$ puis on verse la solution sur 150 g de glace. On sépare la phase organique et on extrait la phase aqueuse par 2x100 ml de $CH_2Cl_2$. On lave les phases organiques réunies avec une solution saturée de $K_2CO_3$ puis avec de l'eau distillée. On sèche sur $Na_2SO_4$ et on évapore le solvant sous vide. On dissout l'huile obtenue dans un minimum d'éther diéthylique et on ajoute du pentane pour faire précipiter le produit. Le produit pur cristallin est directement filtré (9,76 g ; rendement 93%).
Caractéristiques :
RMN $^1H$ (200 MHz , $CDCl_3$) δ 6,69 (d, J = 3,9 Hz, 1H) ; 5,51 (dd, J = 3,2 Hz, J = 1 Hz 1H) ; 5,40 (dd, J = 10,6 Hz, J = 3,2 Hz, 1H) ; 5,04 (dd, J = 10,6 Hz, J = 3,9 Hz, 1H) ; 4,48 (t, J = 6,5 Hz, 1H) ; 4,17 (m, 2H) ; 2,15 (s, 3H) ; 2,11 (s, 3H) ; 2,09 (s, 3H) ; 2,01 (s, 3H) ;

$T_{fus}$ = 85,3˚C ; $[\alpha]_{289nm}^{20^\circ C} = 219°$ (c = 1 ; $CHCl_3$).

**Exemple 9**

**Préparation du tétraacétyl-(p-formylphénoxy)-β-galactose (composé 3')**

On dissout le tétraacétyl-α-bromogalactose (4,112 g ; 10 mmol) dans 100 ml d'acétonitrile distillé. On ajoute $Ag_2O$ (10,0g ; 43 mmol) et le p-hydroxybenzaldéhyde (1,222 g ; 10 mmol). On laisse le mélange sous agitation pendant 4 h, on évapore le solvant sous vide et on filtre la suspension métallique résultante sur une couche de silice en utilisant

l'acétate d'éthyle comme éluant. On évapore le solvant sous vide et l'huile jaune obtenue est purifiée par colonne de chromatographie sur gel de silice ($Et_2O$). Le produit pur cristallise par évaporation lente du solvant dans les fractions le contenant. On obtient alors 3,04 g (rendement 67%) de cristaux.

Caractéristiques

RMN $^1H$ (200 MHz , $CDCl_3$) δ 9,93 (s, 1H) ; 7,85 (d, J = 8,7 Hz, 2H) ; 7,11 (d, J = 8,7 Hz, 2H) ; 5,47 (m, 2H) ; 5,17 (m, 2H) ; 4,17 (m, 3H) ; 2,19 (s, 3H) ; 2,06 (s, 6H) ; 2,02 (s, 3H) ;

RMN $^{13}C$ (50 MHz, CDCl3) δ 190,60 ; 170,22 ; 170,07 ; 169,97 ; 169,21 ; 161,22 ; 131,73 ; 116,68 ; 98,53 ; 71,27 ; 70,60 ; 68,35 ; 66,71 ; 61,29 ; 20,56 ; 20,48;

$T_{fus}$ = 116.4°C.

**Exemple 10**

**Préparation du tétraacétyl-p-(pyridinyl-hydroxyméthyl)phénoxy)-β-galactose (composé 4')**

On dissout de la 2-bromopyridine (726 mg ; 440 µl, 4,6 mmol) dans 5 ml de THF à température ambiante. On ajoute goutte-à-goutte du chlorure d'isopropyle de magnésium (2,3 ml ; 4,6 mmol ; 2 M dans le THF) sous argon et on laisse le mélange sous agitation pendant 2 heures. On ajoute cette solution à une solution de tétraacétyl-(p-formylphénoxy)-β-galactose (2,07 g ; 4,6 mmol) dans 3 ml de THF à température ambiante. On laisse sous agitation le mélange pendant 18 h puis on ajoute lentement une solution saturée de $NH_4Cl$. On extrait la solution aqueuse résultante avec de l'acétate d'éthyle. On sèche la phase organique sur $Na_2SO_4$ et on l'évapore sous vide. Le brut de réaction est purifié par chromatographie sur gel de silice (éther/pentane 2/1 éther pur). On obtient alors un solide jaune (600 mg, rendement 25%).

Caractéristiques :

RMN $^1H$ (200 MHz, $CDCl_3$) δ 8,57 (d, J = 4,7 Hz, 1H) ; 7,63 (td, J = 7,6 Hz, J = 1,4 Hz, 1H) ; 7,30 (d, J = 8,6 Hz, 2H) ; 7,21 (t, J = 4,7 Hz, 1H) ; 7,13 (d, J = 7,6 Hz, 1H) ; 6,97 (d, J = 8,6 Hz, 2H) ; 5,72 (d, J = 4,2 Hz, 1H) ; 5,43-5,47 (m, 2H) ; 5,19 (d, J = 4,2 Hz, 1H) ; 5,08 (dd, J = 7,9 Hz, J = 4,2 Hz, 1H) ; 5,01 (d, J = 7,9 Hz, 1H) ; 3,97 - 4,22 (m, 3H) ; 2,17 (s, 3H) ; 2,05 (s, 3H) ; 2,03 (s, 3H) ; 2,00 (s, 3H).

**Exemple 11**

**Préparation du tétraacétyl-p-(pyridinylchlorométhyl) phénoxy)-β-galactose (composé 5')**

On dissout le tétraacétyl-p-(pyridinyl hydroxyméthyl)-phénoxy)-β-galactose (600 mg ; 1,1 mmol) dans 15 ml de $CH_2Cl_2$. On ajoute goutte-à-goutte du $SOCl_2$ (143 mg, 88 µl ; 1,2 mmol) et on laisse la solution sous agitation pendant 1 heure. On évapore le solvant sous vide. Le produit est obtenu pur et quantitativement.

Caractéristiques :

RMN $^1H$ (200 MHz , $CDCl_3$) δ 8,64 (d, J = 5,2 Hz, 1H) ; 8,42 (t, J = 7,8 Hz, 1H) ; 8,19 (d, J = 7,8 Hz, 1H) ; 7,81 (t, J = 6,4 Hz, 1H) ; 7,67 (dd, J = 8,6 Hz, J = 3,3, 2H) ; 7,11 (d, J = 2,1 Hz, 1H) ; 7,01 (d, J = 8,6 Hz, 2H) ; 5,44 - 5,51 (m, 2H) ; 5,12 (d, J = 2,9 Hz, 1H) ; 5,04 (d, J = 7,9 Hz, 1H) ; 4,01 - 4,20 (m, 3H) ; 2,17 (s, 3H) ; 2,06 (s, 3H) ; 2,03 (s, 3H) ; 2,00 (s, 3H).

**Exemple 12**

**Préparation du 1,4-dipyridinylméthyl-7-(pyridinyl(p-tétraacétyl-β-galactophényl) méthyl)- 1,4,7-triazacyclononane (composé 6')**

On mélange du 1,4-dipyridinylméthyl-1,4,7-triazacyclononane (168 mg ; 0,5 mmol), du tétraacétyl-p-(pyridinylchlorométhyl)phénoxy)-β-galactose (356 mg ; 0,6 mmol) et du $K_2CO_3$ (environ 1, 5g ; 11 mmol) dans 10 ml d'acétonitrile. La suspension résultante est laissée sous agitation une nuit à 50°C sous argon. Après retour à la température ambiante, on filtre et on lave le précipité par 2x5 ml d'acétonitrile. On évapore le solvant du filtrat et on obtient une huile jaune que l'on purifie par chromatographie sur gel de silice ($CH_2Cl_2$/MeOH 10/1 à $CH_2Cl_2$/MeOH/$NH_4OH$ 4/1/0,1) de manière à obtenir le produit pur (327 mg, rendement 74%).

Caractéristiques :

RMN $^1H$ (200 MHz , $CDCl_3$) δ 8,48 (t, J = 4,4 Hz, 3H) ; 7,63 (td, J = 7,6 Hz, J = 1,6 Hz, 3H) ; 7,36 (d, J = 8,6 Hz, 2H); 7,13 (m, 3H) ; 6,89 (d, J = 8,6 Hz, 2H) ; 5,42 (m, 2H) ; 5,06 (dd, J = 7,9 Hz, J = 4,2 Hz, 1H) ; 4,98 (d, J = 7,9 Hz, 1H) ; 4,78 (s, 1H) ; 3,97 - 4,22 (m, 3H) ; 3,79 (s, 4H) ; 2,96 (s, 4H) ; 2,84 (d, J = 8,6 Hz, 4H) ; 2,16 (s, 3H) ; 2,03 (s, 3H) ; 2,02 (s, 3H) ; 2,00 (s, 3H).

RMN $^{13}C$ (50 MHz, $CDCl_3$) δ 179,29 (C) ; 179,21 (C) ; 170,09 (C) ; 169,33 (C) ; 162,98 (C) ; 160,40 (C) ; 155,82 (C); 148,88 (CH) ; 137,19 (C) ; 136,42 (CH) ; 136,24 (CH) ; 129,79 (CH) ; 123,16 (CH) ; 122,64 (CH) ; 121,80 (CH) ; 116,73

(CH) ; 99,50 (CH) ; 70,84 (CH) ; 68, 61 (CH) ; 66, 82 (CH) ; 64,63 ($CH_2$) ; 61,26 ($CH_2$) ; 55,78 ($CH_2$) ; 55,52 ($CH_2$) ; 53,99 ($CH_2$) ; 20,62 ($CH_3$).

**Exemple 13**

**Préparation du 1,4-dipyridinylméthyl-7-(pyridinyl(p-β-galactophényl)méthyl)-1,4,7-triazacyclononane**

On dissout le 1,4-dipyridinylméthyl-7-(pyridinyl(p-tétraacétyl-β-galactophényl)méthyl)-1,4,7-triazacyclononane (124 mg ; 0,15 mmol) dans 2 ml de méthanol. On ajoute du méthanolate de sodium (9 mg ; 0,15 mmol). On laisse le mélange réactionnel sous agitation pendant 4 h sous argon, période après laquelle le substrat est complètement consommé selon le LCMS. On évapore le solvant sous vide puis on ajoute $CHCl_3$ (2 ml) et un précipité apparaît. On filtre la solution et on lave le solide à l'aide de chloroforme. On évapore le solvant sous vide et on obtient le produit sous forme d'une huile jaune (78 mg, rendement 79%).

**Exemple 14**

**Préparation de [Fe(1,4-dipyridinylméthyl-7-(pyridinyl(p-β-galactophényl)méthyl)-1,4,7-triazacyclononane)]$^2$ $(BF)_4^-$**

On ajoute une solution de bis(tétrafluoroborate) de fer (II) (6 $H_2O$) (68 mg ; 0,18 mmol) dans du méthanol dégazé (1 ml) via une canule à une solution de 1,4-dipyridinylméthyl-7-(pyridinyl(p-β-galactophényl)méthyl)-1,4,7-triazacyclononane (139 mg ; 0,21 mmol) dans 0,5 ml de méthanol dégazé sous argon. Une couleur brun foncé apparaît instantanément. On laisse le mélange sous agitation pendant 10 minutes à température ambiante puis on évapore le solvant sous vide. On purifie le brut de réaction par deux filtrations successives sur colonne de silice phase inverse $C^{18}$ en utilisant l'eau comme éluant, pour obtenir le complexe pur sous forme d'un solide jaune après lyophilisation (103 mg, rendement 65%).

**Revendications**

1. Agent de contraste pour l'imagerie par résonance magnétique comprenant un ligand chélatant et un ion de métal de transition, ledit ligand portant un substituant capable de réagir par voie chimique ou biochimique avec une substance cible en provoquant un changement de l'état de spin, **caractérisé en ce qu'**il possède :

   a) soit la formule (I)

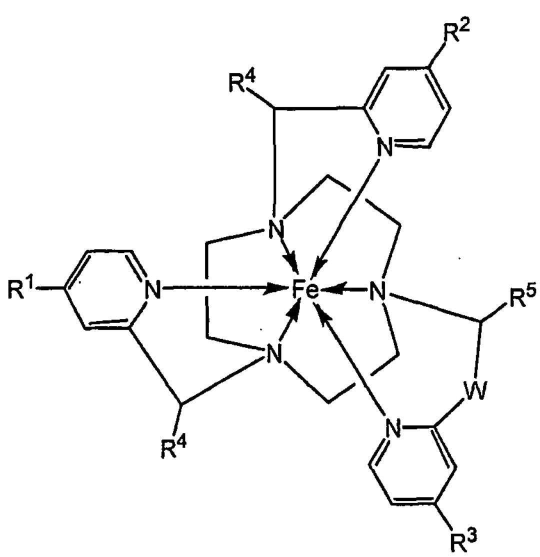

(I)

dans laquelle :

- $R^4$ représente un atome d'hydrogène ou un groupe alkyle de 1 à 4 atomes de carbone, linéaire ou ramifié, ou un radical aryle ;
- W représente un groupe $-(CH_2)_m-$, m étant égal à 0 ou à 1 ;
- $R^5$ représente un groupe capable de réagir avec la substance cible pour provoquer une coupure entre l'atome N du macrocycle et le carbone portant ledit groupe $R^5$, ou représente un atome d'hydrogène, étant entendu que :

i) lorsque $R^5$ représente un groupe capable de réagir avec la substance cible pour provoquer une coupure entre l'atome N du macrocycle et le carbone portant ledit groupe $R^5$ (complexes Ia), les substituants $R^1$ à $R^3$, désignés par $R^i$, représentent, indépendamment les uns des autres, un atome d'hydrogène ou un groupe choisi pour ajuster les propriétés de solubilité, de dispersibilité dans les milieux biologiques, et de moment magnétique du complexe, l'un des $R^i$ pouvant encore représenter un groupe capable de subir une modification électronique du groupement pyridyle voisin par réaction avec la substance cible ;
ii)lorsque $R^5$ représente un atome d'hydrogène (complexes Ib), l'un des groupes $R^i$ représente un groupe capable de subir une modification électronique du groupement pyridyle voisin par réaction avec la substance cible, les groupes $R^i$ restant pouvant représenter, indépendamment les uns des autres, un atome d'hydrogène

ou être choisis pour ajuster les propriétés de solubilité, de dispersibilité dans les milieux biologiques, et de moment magnétique du complexe ;

b) soit la formule (II)

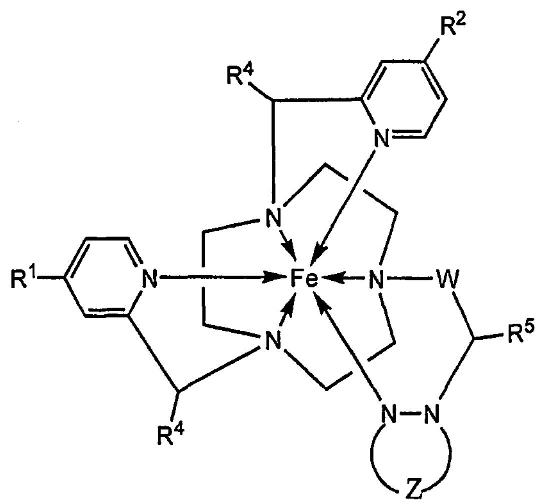

(II)

dans laquelle :

- $R^1$ et $R^2$, désignés par $R^i$, représentent, indépendamment les uns des autres, un atome d'hydrogène ou un groupe choisi pour ajuster les propriétés de solubilité, de dispersibilité dans les milieux biologiques et de moment magnétique du complexe ;
- $R^4$ et W sont tels que définis précédemment ;
- $R^5$ représente un groupe $(-C_6X_4-Y-R^6)$, dans lequel X est un groupement électroattracteur, Y est un hétéroatome choisi parmi un atome d'oxygène, un atome d'azote ou un atome de soufre, et $R^6$ représente un groupe susceptible de subir un clivage par une glycosidase, ou un groupe aminoacyle susceptible de subir un clivage par une aminopeptidase, ou un groupe acyle susceptible de subir un clivage par une estérase ou une lipase, ou un groupe susceptible de subir une transformation retro-aldol par une aldolase naturelle,

ou un groupe susceptible de subir une transformation par une phosphatase ;

- Z est un groupement divalent qui forme avec les deux atomes d'azote qui le portent un cycle aromatique benzotriazole, triazole, tétrazole, pyrazole, ledit cycle aromatique pouvant porter un substituant $NO_2$.

**2.** Agent de contraste de formule (II) selon la revendication 1, **caractérisé en ce que :**

- $R^6$ représente un groupe β-galactosyle, un groupe β-glucuronyle, un groupe L-prolyle, un groupe L-leucyle, un groupe $-COC_5H_{11}$, un groupe de type α,β-dihydroxycétone

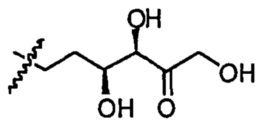

ou un groupe phosphoryle ;

- les groupements X représentent, indépendamment les uns des autres, un atome d'hydrogène, un atome de fluor ou un groupe $NO_2$.

**3.** Agent de contraste selon la revendication 1 répondant à la formule (Ia), **caractérisé en ce que** les groupes $R^i$ représentent, indépendamment les uns des autres, un atome d'hydrogène, un groupe électroattracteur choisi parmi les groupes $-COOR^7$ dans lesquels $R^7$ représente un groupe alkyle ayant de 1 à 4 atomes de carbone, le groupe $-NO_2$, un atome d'halogène et le groupe $-CH_2-COO^-$, ou bien l'un des trois groupes $R^i$ représente un groupe -CH (OH)CH(COOH)$NH_2$, les $R^i$ restant étant alors tels que définis ci-dessus.

**4.** Agent de contraste selon la revendication 3 répondant à la formule Ia, **caractérisé en ce que** $R^5$ répond à la formule $-E-R^6$, dans laquelle E est un groupe espaceur capable de s'auto-effondrer en cas de coupure de la liaison $E-R^6$ et $R^6$ est un groupe capable de réagir avec un composé chimique ou un composé biochimique en déclenchant une succession de coupures provoquant l'élimination de la pyridine-2-carbaldéhyde.

**5.** Agent de contraste selon la revendication 4,
**caractérisé en ce que :**

- $R^6$ représente un groupe susceptible de subir un clivage par une glycosidase ou un groupe aminoacyle susceptible de subir un clivage par une aminopeptidase ou un groupe acyle susceptible de subir un clivage par une esterase ou une lipase, ou un groupe susceptible de subir une transformation retro-aldol par une aldolase naturelle, ou un groupe susceptible de subir une transformation par une phosphatase, par exemple un groupe phosphoryle ;
- E est un groupe $-O-CO-(CR'_2)_n-Y-$ dans lequel les groupes R' représentent, indépendamment les uns des autres, un atome d'hydrogène, un groupe méthyle ou deux R' portés par deux atomes de carbone voisins forment ensemble un cycle aliphatique ou aromatique à 5 ou 6 chaînons porté par les deux atomes de carbone voisins;
- n est un entier égal à 3 ou 4 ;
- Y est un hétéroatome choisi parmi un atome d'oxygène, un atome d'azote ou un atome de soufre.

**6.** Agent de contraste selon la revendication 5,
**caractérisé en ce que** $R^6$ représente un groupe β-galactosyle, un groupe β-glucuronyle, un groupe L-prolyle, un groupe L-leucyle, un groupe $-COC_5H_{11}$, un groupe de type α,β-dihydroxycétone

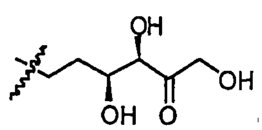

ou un groupe phosphoryle ;

**7.** Agent de contraste selon la revendication 5 ou 6, **caractérisé en ce que** le cycle aromatique à 5 ou 6 chaînons

est choisi parmi les groupes phényle, pyridyle, pyrimidinyle, pyrazinyle, indoyle, indazolyle, furyle et thiényle, lesdits groupes pouvant porter différents substituants choisis parmi les groupes méthyle, chloro, fluoro, nitro, méthoxy, carboxy et acétamido, et le cycle aliphatique à 5 ou 6 chaînons est un groupe cyclohexyle ou un groupe cyclopentyle.

**8.** Agent de contraste selon l'une quelconque des revendications 5 à 7, **caractérisé en ce que** E répond à l'une des formules suivantes :

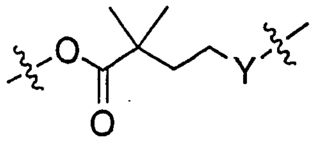

E1

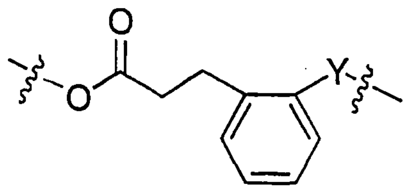

E2

**9.** Agent de contraste selon la revendication 1 ou 3 répondant à la formule Ia, **caractérisé en ce que :**

- $R^5$ répond à la formule $-C_6X_4$-Y-$R^6$ dans laquelle ($C_6X_4$-Y)est un groupe espaceur capable de s'auto-effondrer en cas de coupure de la liaison - ($C_6X_4$-Y)-$R^6$, et
- $R^6$ est un groupe capable de réagir avec un composé chimique ou un composé biochimique en déclenchant une succession de coupures provoquant l'élimination d'une quinone-méthide.

**10.** Agent de contraste selon la revendication 9,
**caractérisé en ce que :**

- Y est un hétéroatome choisi parmi un atome d'oxygène, un atome d'azote ou un atome de soufre;
- X est un groupement électroattracteur,et
- $R^6$ représente un groupe susceptible de subir un clivage par une glycosidase, ou un groupe aminoacyle susceptible de subir un clivage par une aminopeptidase, ou un groupe acyle susceptible de subir un clivage par une esterase ou une lipase, ou un groupe susceptible de subir une transformation retro-aldol par une aldolase naturelle, ou un groupe susceptible de subir une transformation par une phosphatase, par exemple un groupe phosphoryle.

**11.** Agent de contraste selon la revendication 10,
**caractérisé en ce que :**

- $R^6$ représente un groupe β-galactosyle, un groupe β-glucuronyle, un groupe L-prolyle, un groupe L-leucyle, un groupe $-COC_5H_{11}$, un groupe de type α,β-dihydroxycétone

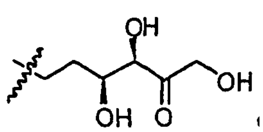

ou un groupe phosphoryle ;

- les groupements X représentent, indépendamment les uns des autres, un atome d'hydrogène, un atome de fluor ou un groupe $NO_2$.

**12.** Agent de contraste selon l'une quelconque des revendications 9 à 11, **caractérisé en ce que** ($C_6X_4$-Y) répond à l'une des formules suivantes :

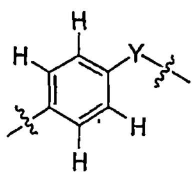 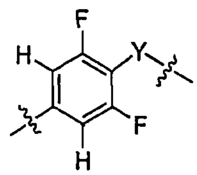 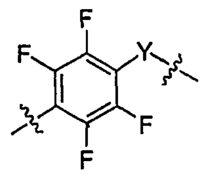 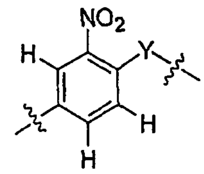

**13.** Agent de contraste selon la revendication 1 répondant à la formule Ib, **caractérisé en ce que** l'un des trois groupes $R^i$ représente un groupe $-CH(OH)CH(COOH)NH_2$ et les groupes $R^i$ restant représentent, indépendamment les uns des autres, un atome d'hydrogène, un atome d'halogène, ou un groupe choisi parmi les groupes $-COOR^7$ dans lesquels $R^7$ peut représenter un groupe alkyle ayant de 1 à 4 atomes de carbone, le groupe $-NO_2$, le groupe -CHO.

**14.** Agent de contraste selon la revendication 1 ou 2 répondant à la formule (II), **caractérisé en ce que** ($C_6X_4$-Y) répond à l'une des formules suivantes :

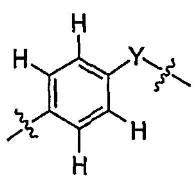 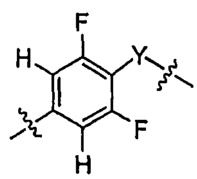 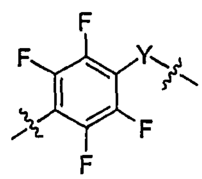 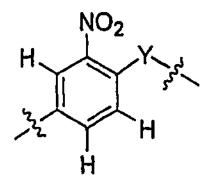

**15.** Composition pharmaceutique **caractérisée en ce qu'**elle comprend un agent de contraste selon l'une quelconque des revendications 1 à 14.

**16.** Utilisation d'un agent de contraste selon l'une quelconque des revendications 1 à 14 pour la préparation d'une composition pharmaceutique destinée au diagnostic par imagerie par résonance magnétique.

**17.** Composition pharmaceutique selon la revendication 15, destinée à la détermination de la distribution tissulaire de β-galactosidase ou de β-glucuronidase, **caractérisée en ce que** l'agent de contraste est tel que défini dans l'une des revendications 4 ou 9, $R^6$ représentant respectivement un groupe β-galactosyle ou un groupe β-glucuronyle.

**18.** Composition pharmaceutique selon la revendication 15, destinée à la détermination de la distribution tissulaire d'aminopeptidases, de lipase, de transaldolases, et de phosphatases, **caractérisée en ce que** l'agent de contraste est tel que défini dans l'une des revendications 4 ou 9, $R^6$ représentant un groupe L-leucyle, un groupe $-COC_5H_{11}$, un groupe de type α,β-dihydroxyacétone

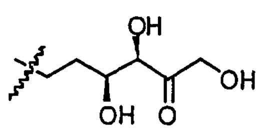 ,

un groupe prolyle, ou un groupe phosphoryle.

**19.** Composition pharmaceutique selon la revendication 15, destinée à la détermination de la distribution tissulaire de *L*-thréonine aldolase, **caractérisée en ce que** l'agent de contraste est tel que défini dans la revendication 13.

**20.** Composé de formule (4),

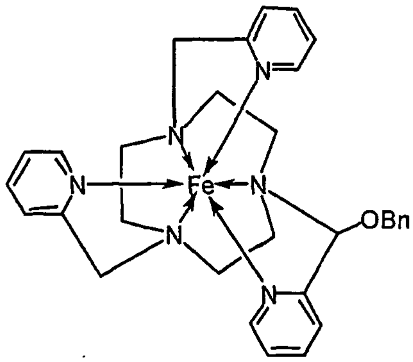

(4)

utile en tant qu'intermédiaire de synthèse.

**21.** Procédé de préparation d'un complexe de formule (Ia), dans laquelle $R^5$ et $R^i$ sont tels que définis dans l'une quelconque des revendications 4 à 8, **caractérisé en ce que** l'on procède par couplage d'un composé (4) selon la revendication 20, préalablement hydrogéné, avec un chlorure d'acyle de formule $Cl-CO-(CR'_2)_n-Y-R^6$ en présence d'une base pour obtenir le composé dans lequel le groupe $R^6$ est éventuellement sous forme protégée, éventuellement suivie d'une déprotection pour obtenir le complexe de formule (Ia-1).

**22.** Procédé de préparation d'un complexe de formule (Ia-1) dans laquelle $R^5$ est un groupe $-O-CO-C(CH_3)_2-(CH_2)_2-OR^6$, $R^6$ est un groupe β-galactosyle penta-acétylé et les autres substituants $R^i$ et $R^4$ sont des atomes d'hydrogène, **caractérisé en ce qu'**il comprend une étape consistant à faire réagir un composé (4) selon la revendication 21, préalablement hydrogéné, avec un composé de formule (6) ci-dessous

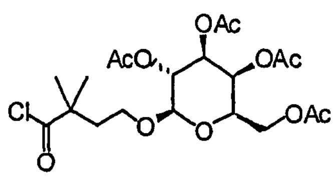

(6)

en présence d'une base, pour obtenir le complexe dans lequel le groupe galactosyle est sous forme protégée, suivie d'une déprotection pour obtenir le complexe de formule (Ia-1).

**23.** Composé de formule (9)

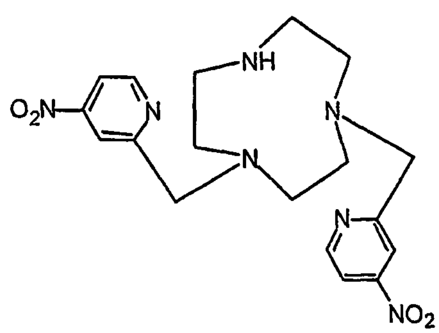

(9)

utile en tant qu'intermédiaire de synthèse.

**24.** Procédé de préparation d'un complexe de formule (Ia-2) dans laquelle $R^5$ est un groupe $C_6H_4-O-R^6$, $R^6$ étant un groupe β-galactosyle penta-acétylé et les autres substituants $R^i$ et $R^4$ étant des atomes d'hydrogène **caractérisé**

**en ce qu'**il comprend une étape consistant à faire réagir un composé de formule (2') ci-dessous

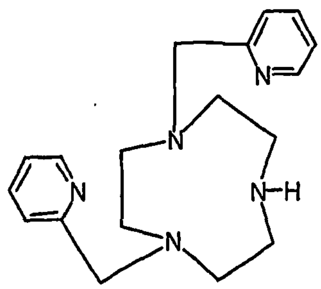

(2')

avec un composé de formule (5') ci-dessous

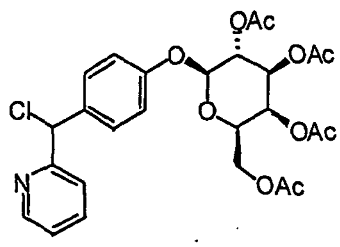

(5')

pour obtenir le composé de formule (6') ci-dessous dans lequel le groupe galactosyle est sous forme protégée,

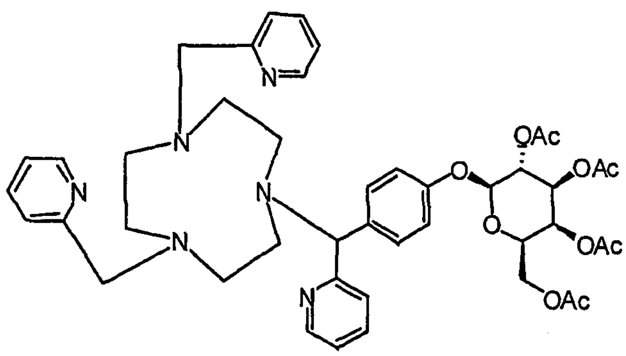

(6')

suivie d'une étape de déprotection et d'une étape de traitement par $Fe(BF_4)_2$ pour obtenir le complexe de formule (Ia-2).

**25.** Procédé de préparation d'un complexe de formule (Ib) dans laquelle $R^5$ est un atome d'hydrogène et l'un des substituants $R^i$ est un groupe $CH(OH)CH(COOH)NH_2$, **caractérisé en ce qu'**il comprend une étape consistant à faire réagir un composé de formule (9) selon la revendication 23, avec un composé de formule (8) ci-dessous

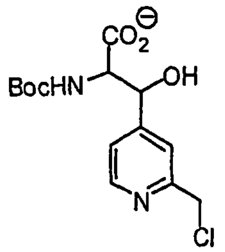

(8)

suivie d'une étape de déprotection du groupement amine primaire et d'une transformation en complexe de fer pour obtenir le complexe de formule (Ib).

**26.** Procédé de préparation d'un complexe de formule (II) dans laquelle $R^5$ est un groupement $-C_6H_4$-O-β-galactosyle, les deux atomes d'azote et le groupement Z constituent ensemble une unité benzotriazole, et $R^1$, $R^2$ et $R^4$ représentent des atomes d'hydrogène, **caractérisé en ce qu'**il comprend une étape consistant à faire réagir un composé de formule (1") ci-dessous

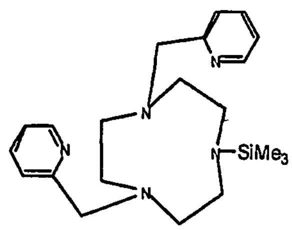

(1")

avec un composé de formule (2") ci-dessous

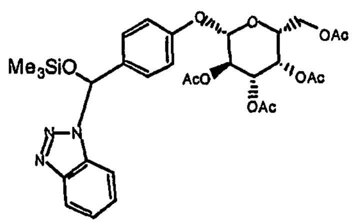

(2")

pour obtenir le composé de formule (3") ci-dessous dans lequel le groupe galactosyle est sous forme protégée,

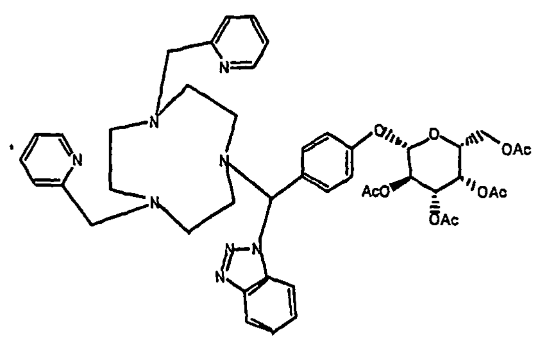

(3")

suivie d'une étape de traitement par $Fe(BF_4)_2$, suivie d'une étape de déprotection pour obtenir le complexe de

formule (II).

**27.** Utilisation selon la revendication 16, d'un agent de contraste répondant à la formule(Ia), dans laquelle $R^5$ représente un groupe -E-$R^6$ ou un groupe ($C_6X_4$-Y-$R^6$), $R^6$ représentant respectivement un groupe β-galactosyle ou un groupe β-glucuronyle, et les groupes E, ($C_6X_4$-Y), $R^4$ et $R^i$ sont tels que définis à l'une quelconque des revendications 3 à 12, pour la préparation d'une composition pharmaceutique destinée à la détermination de la distribution tissulaire de β-galactosidase ou de β-glucuronidase.

**28.** Utilisation selon la revendication 16, d'un agent de contraste répondant à la formule (Ia), dans laquelle $R^5$ représente un groupe E-$R^6$ ou un groupe ($C_6X_4$-Y-$R^6$), $R^6$ représentant un groupe L-leucyle, un groupe -$COC_5H_{11}$, un groupe de type α,β-dihydroxyacétone

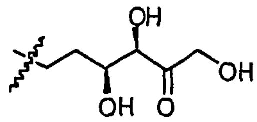

un groupe prolyle ou un groupe phosphoryle, et les groupes E, ($C_6X_4$-Y), $R^4$ et $R^i$ sont tels que définis à l'une quelconque des revendications 3 à 12, pour la préparation d'une composition pharmaceutique destinée à la détermination de la distribution tissulaire d'aminopeptidase, de lipase, de transaldolase ou de phosphatase.

**29.** Utilisation selon la revendication 16, d'un agent de contraste répondant à la formule (Ib) selon la revendication 13 pour la préparation d'une composition pharmaceutique destinée à la détermination de la distribution tissulaire de *L*-thréonine aldolase.

**30.** Méthode d'imagerie par résonance magnétique d'une cellule ou d'un tissu auquel un agent de contraste selon l'une quelconque des revendications 1 à 14 a été administré.

## Claims

**1.** A contrast agent for magnetic resonance imaging comprising a chelating ligand and a transition metal ion, said ligand carrying a substituent capable of reacting chemically or biochemically with a target substance thereby causing a change in the spin state, **characterized in that** it has:

a) either the formula (I):

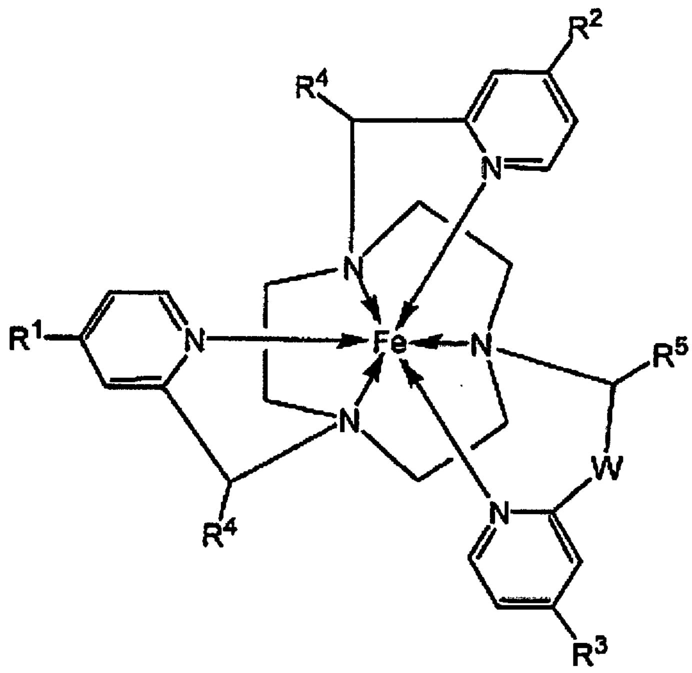

(I)

in which:

- $R^4$ represents a hydrogen atom or a linear or branched alkyl group containing 1 to 4 carbon atoms or an aryl radical;
- W represents a $-(CH_2)_m-$ group, m being equal to 0 or to 1;
- $R^5$ represents a group which is capable of reacting with the target substance to cause cleavage between the N atom of the macrocycle and the carbon carrying said group $R^5$, or represents a hydrogen atom, it being understood that:

  i) when $R^5$ represents a group which is capable of reacting with the target substance to cause cleavage between the N atom of the macrocycle and the carbon carrying said group $R^5$ (complexes Ia), substituents $R^1$ to $R^3$, denoted by $R^i$, independently of each other represent a hydrogen atom or a group which is selected in order to adjust the properties of solubility and of dispersibility in biological media and of the magnetic moment of the complex, one of the $R^i$ groups optionally representing a group which is capable of undergoing electronic modification of the neighboring pyridyl group by reaction with the target substance;
  ii) when $R^5$ represents a hydrogen atom (complexes Ib), one of the $R^i$ groups represents a group which is capable of undergoing electronic modification of the neighboring pyridyl group by reaction with the target substance, the remaining $R^i$ groups optionally representing, independently of each other, a hydrogen atom or being selected in order to adjust the properties of solubility and of dispersibility in biological media and of the magnetic moment of the complex;

b) or the formula (II):

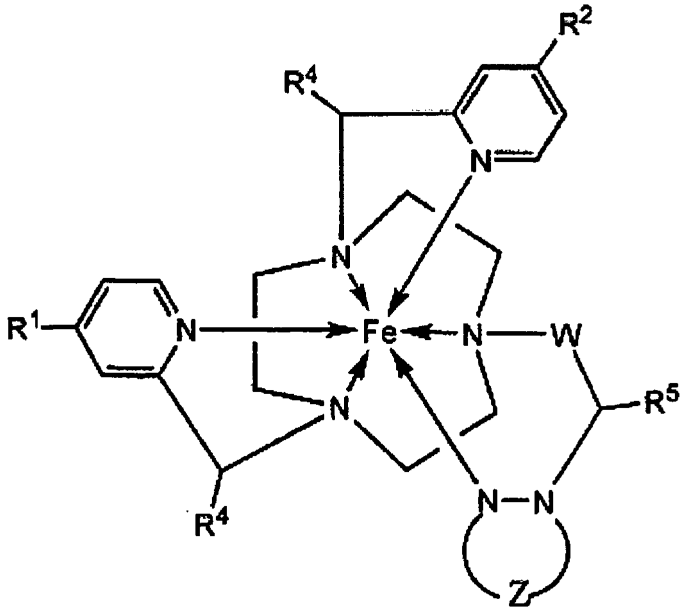

(II)

in which:

- $R^1$ and $R^2$, denoted by $R^i$, independently of each other represent a hydrogen atom or a group selected in order to adjust the properties of solubility and of dispersibility in biological media and of the magnetic moment of the complex;
- $R^4$ and W are as defined above;
- $R^5$ represents a group ($-C_6X_4-Y-R^6$) in which X is an electron-withdrawing group, Y is a heteroatom selected from an oxygen atom, a nitrogen atom and a sulfur atom, and $R^6$ represents a group which is capable of undergoing cleavage by a glycosidase, or an aminoacyl group which is capable of undergoing cleavage by an aminopeptidase, or an acyl group which is capable of undergoing cleavage by an esterase or a lipase, or a group which is capable of undergoing a retrograde aldol reaction with a natural aldolase, or a group which is capable of undergoing transformation by a phosphatase;
- Z is a divalent group which, with the two nitrogen atoms which carry it, forms an aromatic benzotriazole, triazole, tetrazole or pyrazole ring, said aromatic ring possibly carrying an $NO_2$ substituent.

**2.** The contrast agent with formula (II) as claimed in claim 1, **characterized in that**:

- $R^6$ represents a β-galactosyl group, a β-glucuronyl group, an L-prolyl group, an L-leucyl group, a $-COC_5H_{11}$ group, a group of the α,β-dihydroxyketone type

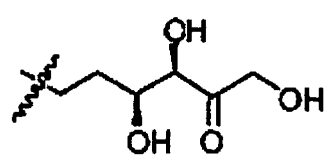

or a phosphoryl group;
- the X groups independently of each other represent a hydrogen atom, a fluorine atom or an $NO_2$ group.

**3.** The contrast agent as claimed in claim 1 having formula (Ia), **characterized in that** the $R^i$ groups independently of each other represent a hydrogen atom, an electron-withdrawing group which is selected from $-COOR^7$ groups in which $R^7$ represents an alkyl group containing 1 to 4 carbon atoms, the $-NO_2$ group, a halogen atom and the

$-CH_2-COO^-$ group, or one of the three $R^i$ groups represents a $-CH(OH)CH(COOH)NH_2$ group, the remaining $R^i$ groups then being as defined above.

4. The contrast agent as claimed in claim 3 having formula Ia, **characterized in that** $R^5$ corresponds to the formula $-E-R^6$ in which E is a spacer group which is capable of self-collapsing in the event of cleavage of the $E-R^6$ bond and $R^6$ is a group which is capable of reacting with a chemical compound or a biochemical compound, triggering a sequence of cleavages causing the elimination of pyridine-2-carbaldehyde.

5. The contrast agent as claimed in claim 4, **characterized in that:**

- $R^6$ represents a group which is capable of undergoing cleavage by a glycosidase, or an aminoacyl group which is capable of undergoing cleavage by an aminopeptidase, or an acyl group which is capable of undergoing cleavage by an esterase or a lipase, or a group which is capable of undergoing a retrograde aldol reaction with a natural aldolase, or a group which is capable of undergoing transformation by a phosphatase, for example a phosphoryl group;
- E is an $-O-CO-(CR'_2)_n-Y-$ group in which the R' groups independently of each other represent a hydrogen atom or a methyl group, or two R' groups carried by two neighboring carbon atoms together form a 5- or 6-membered aliphatic or aromatic ring carried by the two neighboring carbon atoms;
- n is an integer equal to 3 or 4;
- Y is a heteroatom selected from an oxygen atom, a nitrogen atom and a sulfur atom.

6. The contrast agent as claimed in claim 5, **characterized in that** $R^6$ represents a β-galactosyl group, a β-glucuronyl group, an L-prolyl group, an L-leucyl group, a-$COC_5H_{11}$ group, a group of the α,β-dihydroxyketone type

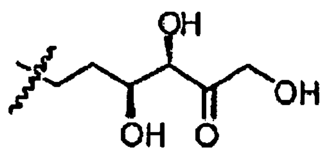

or a phosphoryl group.

7. The contrast agent as claimed in claim 5 or claim 6, **characterized in that** the 5- or 6-membered aromatic ring is selected from the phenyl, pyridyl, pyrimidinyl, pyrazinyl, indoyl, indazolyl, furyl and thienyl groups, said groups possibly carrying various substituents selected from the methyl, chloro, fluoro, nitro, methoxy, carboxyl and acetamido groups, and the 5- or 6-membered aliphatic ring is a cyclohexyl group or a cyclopentyl group.

8. The contrast agent as claimed in any one of claims 5 to 7, **characterized in that** E corresponds to one of the following formulae:

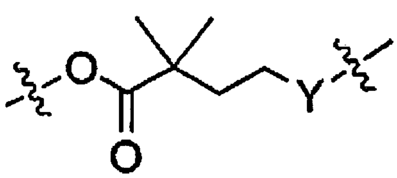

E1

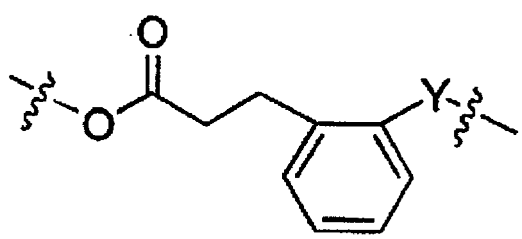

E2

9. The contrast agent as claimed in claim 1 or claim 3 having formula Ia, **characterized in that:**

- $R^5$ corresponds to the formula $-C_6X_4-Y-R^6$ in which $(C_6X_4-Y)$ is a spacer group which is capable of self-collapsing in the event of cleavage of the -($C_6X_4$-Y)-$R^6$ bond, and

- $R^6$ is a group which is capable of reacting with a chemical compound or a biochemical compound, triggering a sequence of cleavages causing elimination of a quinone methide.

**10.** The contrast agent as claimed in claim 9, **characterized in that:**

- Y is a heteroatom selected from an oxygen atom, a nitrogen atom and a sulfur atom;
- X is an electron-withdrawing group; and
- $R^6$ represents a group which is capable of undergoing cleavage by a glycosidase, or an aminoacyl group which is capable of undergoing cleavage by an aminopeptidase, or an acyl group which is capable of undergoing cleavage by an esterase or a lipase, or a group which is capable of undergoing a retrograde aldol reaction with a natural aldolase, or a group which is capable of undergoing transformation by a phosphatase, for example a phosphoryl group.

**11.** The contrast agent as claimed in claim 10, **characterized in that:**

- $R^6$ represents a β-galactosyl group, a β-glucuronyl group, an L-prolyl group, an L-leucyl group, a -$COC_5H_{11}$ group, a group of the α,β-dihydroxyketone type

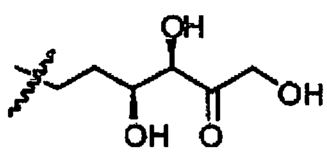

or a phosphoryl group;

- the X groups independently of each other represent a hydrogen atom, a fluorine atom or an $NO_2$ group.

**12.** The contrast agent as claimed in any one of claims 9 to 11, **characterized in that** ($C_6X_4$-Y) corresponds to one of the following formulae:

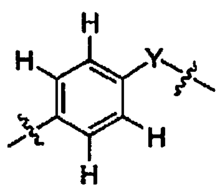
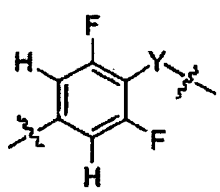
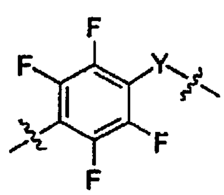
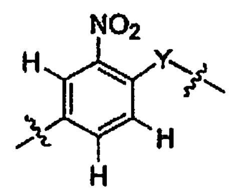

**13.** The contrast agent as claimed in claim 1 having formula Ib, **characterized in that** one of the three $R^i$ groups represents a -$CH(OH)CH(COOH)NH_2$ group and the remaining $R^i$ groups independently of each other represent a hydrogen atom, a halogen atom or a group which is selected from -$COOR^7$ groups in which $R^7$ can represent an alkyl group containing 1 to 4 carbon atoms, the -$NO_2$ group or the -CHO group.

**14.** The contrast agent as claimed in claim 1 or claim 2 having formula (II), wherein ($C_6X_4$-Y) corresponds to one of the following formulae:

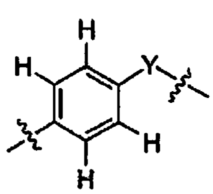
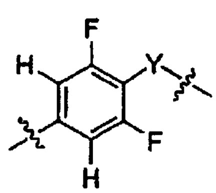
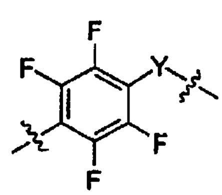
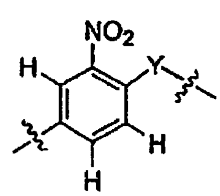

**15.** A pharmaceutical composition, **characterized in that** it comprises a contrast agent as claimed in any one of claims 1 to 14.

**16.** Use of a contrast agent as claimed in any one of claims 1 to 14 in preparing a pharmaceutical composition intended for diagnosis by magnetic resonance imaging.

**17.** The pharmaceutical composition as claimed in claim 15, intended for determining the tissue distribution of β-galactosidase or of β-glucuronidase, **characterized in that** the contrast agent is as defined in either of claims 4 or 9, $R^6$ representing a β-galactosyl group or a β-glucuronyl group respectively.

**18.** The pharmaceutical composition as claimed in claim 15, intended for determining the tissue distribution of aminopeptidases, lipases, transaldolases and phosphatases, **characterized in that** the contrast agent is as defined in either of claims 4 or 9, $R^6$ representing an L-leucyl group, a $-COC_5H_{11}$ group, a group of the α,β-dihydroxyacetone type

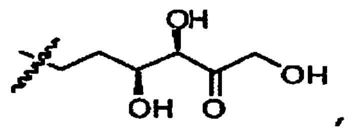

a prolyl group or a phosphoryl group.

**19.** The pharmaceutical composition as claimed in claim 15, intended for determining the tissue distribution of L-threonine aldolase, **characterized in that** the contrast agent is as defined in claim 13.

**20.** A compound with formula (4):

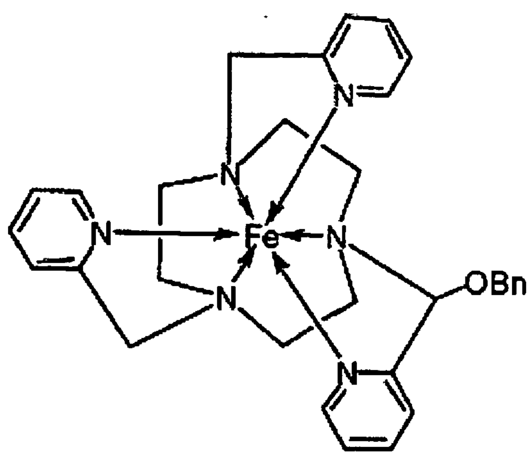

(4)

for use as a synthetic intermediate.

**21.** A process for preparing a complex with formula (Ia) in which $R^5$ and $R^i$ are as defined in any one of claims 4 to 8, **characterized in that** coupling of a compound (4) as claimed in claim 20, which has been hydrogenated, is carried out with an acyl chloride with formula $Cl-CO-(CR'_2)_n-Y-R^6$ in the presence of a base to produce the compound in which the $R^6$ group is optionally in the protected form, optionally followed by deprotection to produce the complex with formula (Ia-1).

**22.** A process for preparing a complex with formula (Ia-1) in which $R^5$ is an $-O-CO-C(CH_3)_2-(CH_2)_2-OR^6$ group, $R^6$ is a penta-acetylated β-galactosyl group and the other $R^1$ and $R^4$ substituents are hydrogen atoms, **characterized in that** it comprises a stage which consists in reacting a compound (4) as claimed in claim 21, which has been

hydrogenated, with a compound with formula (6) below:

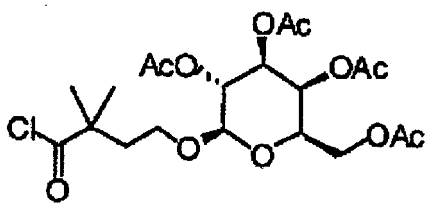

(6)

in the presence of a base to produce the complex in which the galactosyl group is in the protected form, followed by deprotection to produce the complex with formula (Ia-1).

**23.** A compound with formula (9):

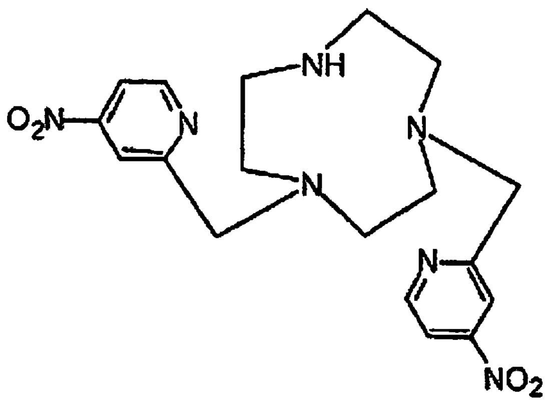

(9)

for use as a synthetic intermediate.

**24.** A process for preparing a complex with formula (Ia-2) in which $R^5$ is a $C_6H_4$-O-$R^6$ group, $R^6$ being a penta-acetylated β-galactosyl group and the other $R^i$ and $R^4$ substituents being hydrogen atoms, **characterized in that** it comprises a stage which consists in reacting a compound with formula (2') below:

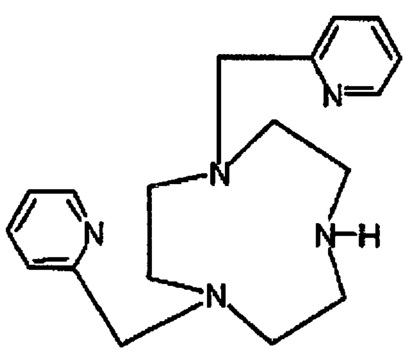

(2')

with a compound with formula (5') below:

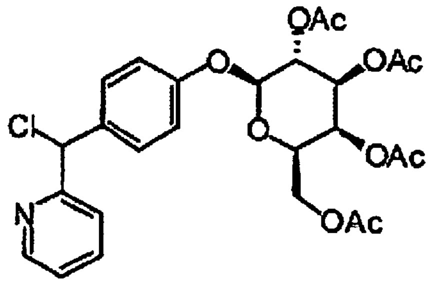

(5')

to produce the compound with formula (6') below in which the galactosyl group is in the protected form:

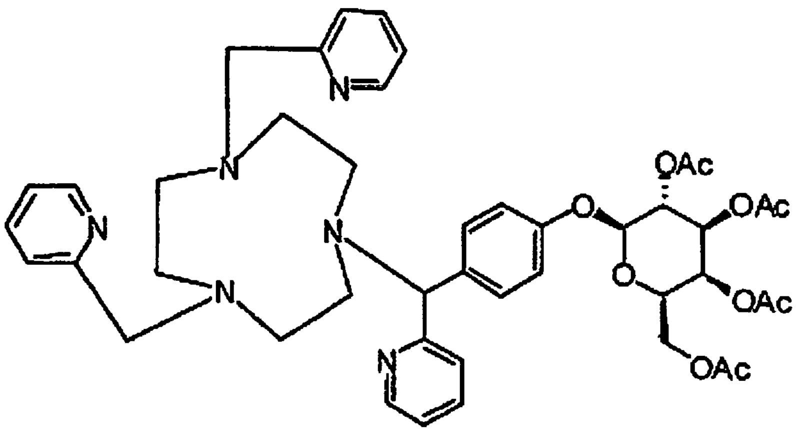

(6')

followed by a stage for deprotection and by a stage for treatment with $Fe(BF_4)_2$ to produce the complex with formula (Ia-2).

**25.** A process for preparing a complex with formula (Ib) in which $R^5$ is a hydrogen atom and one of the $R^i$ substituents is a $CH(OH)CH(COOH)NH_2$ group, **characterized in that** it comprises a stage which consists in reacting a compound with formula (9) as claimed in claim 23 with a compound with formula (8) below:

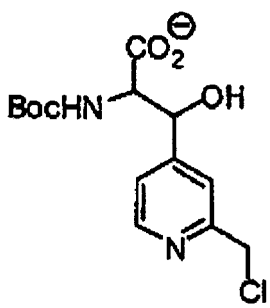

(8)

followed by a stage for deprotection of the primary amine group and conversion into an iron complex to produce the complex with formula (Ib).

**26.** A process for preparing a complex with formula (II) in which $R^5$ is a $-C_6H_4$-O-β-galactosyl group, the two nitrogen atoms and the Z group together constitute a benzotriazole unit and $R^1$, $R^2$ and $R^4$ represent hydrogen atoms,

**characterized in that** it comprises a stage which consists in reacting a compound with formula (1") below:

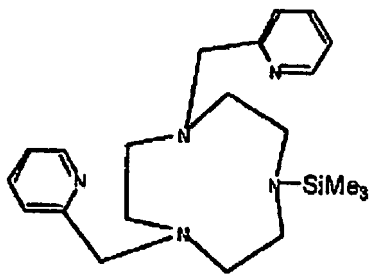

(1")

with a compound with formula (2") below:

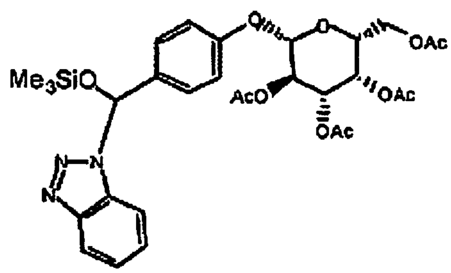

(2")

to produce the compound with formula (3") below in which the galactosyl group is in the protected form:

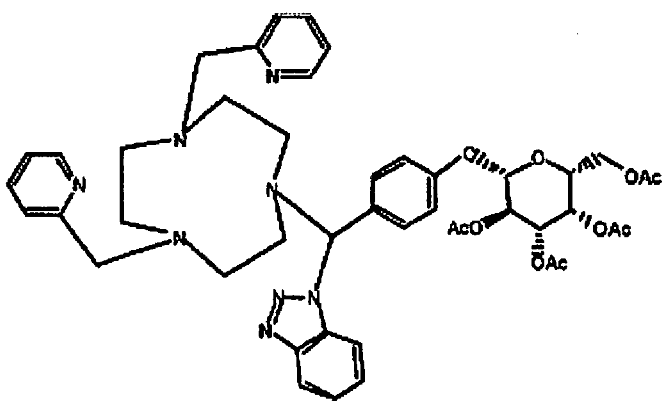

(3")

followed by a stage for treatment with $Fe(BF_4)_2$, followed by a stage for deprotection to produce the complex with formula (II).

**27.** Use as claimed in claim 16 of a contrast agent having formula (Ia) in which $R^5$ represents an -E-$R^6$ group or a ($C_6X_4$-Y-$R^6$ ) group, $R^6$ representing a β-galactosyl group or a β-glucuronyl group respectively, and the E, ($C_6X_4$-Y), $R^4$ and $R^i$ groups are as defined in any one of claims 3 to 12, in preparing a pharmaceutical composition intended for determination of the tissue distribution of β-galactosidase or of β-glucuronidase.

**28.** Use as claimed in claim 16 of a contrast agent having formula (Ia) in which $R^5$ represents an E-$R^6$ group or a ($C_6X_4$-Y-$R^6$) group, $R^6$ representing an L-leucyl group, a -$COC_5H_{11}$ group, a group of the α,β-dihydroxyacetone type

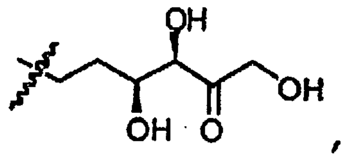

a prolyl group or a phosphoryl group, and the E, ($C_6X_4$-Y), $R^4$ and $R^i$ groups are as defined in any one of claims 3 to 12, in preparing a pharmaceutical composition intended for determination of the tissue distribution of aminopeptidase, lipase, transaldolase or phosphatase.

**29.** Use as claimed in claim 16 of a contrast agent having formula (Ib) as claimed in claim 13 in preparing a pharmaceutical composition intended for determination of the tissue distribution of L-threonine aldolase.

**30.** A method for magnetic resonance imaging of a cell or of a tissue to which a contrast agent as claimed in any one of claims 1 to 14 has been administered.

## Patentansprüche

**1.** Kontrastmittel zur Magnetresonanztomographie, das einen Chelatliganden und ein Übergangsmetallion umfasst, wobei der Ligand einen Substituenten trägt, der in der Lage ist, auf chemischem oder biochemischem Weg mit einer Targetsubstanz zu reagieren, indem er eine Veränderung des Spinzustands herbeiführt, **dadurch gekennzeichnet, dass** es:

a) entweder die Formel (I)

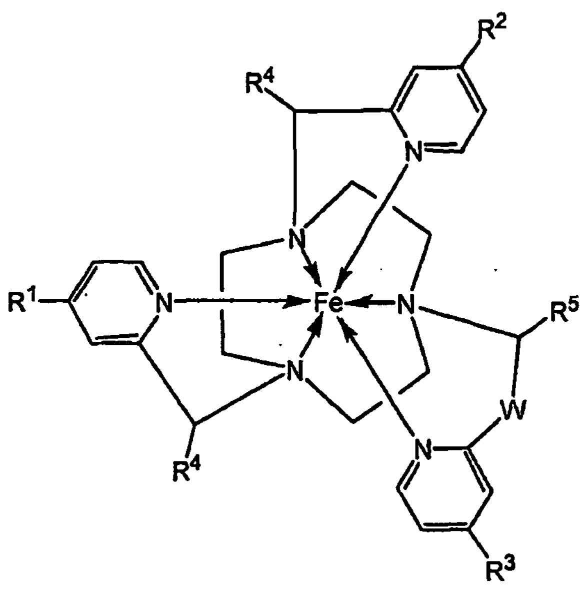

(I)

worin:

- $R^4$ für ein Wasserstoffatom oder eine lineare oder verzweigte Alkylgruppe mit 1 bis 4 Kohlenstoffatomen oder einen Arylrest steht;
- W für eine $(CH_2)_m$-Gruppe steht, wobei m gleich 0 oder 1 ist;
- $R^5$ für eine Gruppe steht, die in der Lage ist, mit der Targetsubstanz zu reagieren, um eine Trennung zwischen dem N-Atom des Makrozyklus und dem Kohlenstoff, der die Gruppe $R^5$ trägt, herbeizuführen, oder für ein Wasserstoffatom steht, wobei gilt:

i) wenn $R^5$ für eine Gruppe steht, die in der Lage ist, mit der Targetsubstanz zur reagieren, um eine Trennung zwischen dem N-Atom des Makrocyclus und dem Kohlenstoff, der die Gruppe $R^5$ trägt, herbeizuführen (Komplexe Ia), stellen die Substituenten $R^1$ bis $R^3$, die mit $R^i$ bezeichnet werden, unabhängig voneinander ein Wasserstoffatom oder eine Gruppe dar, die ausgewählt ist, um die Eigenschaften der Löslichkeit und der Dispergierbarkeit in biologischen Medien und den magnetischen Moment des Komplexes anzupassen, wobei einer der $R^i$-Sustituenten auch für eine Gruppe stehen kann, die in der Lage ist, eine elektronische Veränderung der benachbarten Pyridylgruppe durch Reaktion mit der Targetsubstanz zu erfahren;
ii) wenn $R^5$ für ein Wasserstoffatom steht (Komplexe Ib), steht eine der $R^i$-Gruppen für eine Gruppe, die in der Lage ist, eine elektronische Veränderung der benachbarten Pyridylgruppe durch Reaktion mit der Targetsubstanz zu erfahren, wobei die verbleibenden $R^i$-Gruppen unabhängig voneinander für ein Wasserstoffatom stehen können oder ausgewählt sind, um die Eigenschaften der Löslichkeit und der Dispergierbarkeit in biologischen Medien und den magnetischen Moment des Komplexes anzupassen;

b) oder der Formel (II) aufweist:

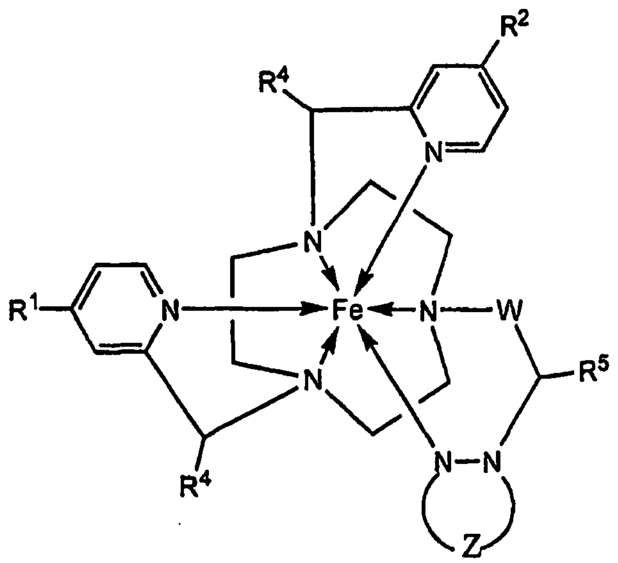

(II)

worin:

- $R^1$ und $R^2$, die mit $R^i$ bezeichnet werden, unabhängig voneinander für ein Wasserstoffatom oder eine Gruppe stehen, die ausgewählt ist, um die Eigenschaften der Löslichkeit und der Dispergierbarkeit in biologischen Medien und den magnetischen Moment des Komplexes anzupassen;
- $R^4$ und W so sind, wie zuvor definiert;
- $R^5$ für eine $(-C_6X_4-Y-R^6)$-Gruppe steht, worin X eine elektronenziehende Gruppe ist, Y ein Heteroatom ist, das aus einem Sauerstoffatom, einem Stickstoffatom oder einem Schwefelatom ausgewählt ist, und $R^6$ für eine Gruppe steht, die eine Spaltung durch eine Glycosidase erfahren kann, oder eine Aminoacylgruppe, die eine Spaltung durch eine Aminopeptidase erfahren kann, oder eine Acylgruppe, die eine Spaltung durch eine Esterase oder eine Lipase erfahren kann, oder eine Gruppe, die eine Retro-Aldol-Umwandlung durch eine natürliche Aldolase erfahren kann, oder eine Gruppe, die eine Umwandlung durch eine Phosphatase erfahren kann;
- Z eine zweiwertige Gruppe ist, die mit den beiden Stickstoffatomen, die sie tragen, einen aromatischen Benzotriazol-, Triazol-, Tetrazol-, Pyrazolring bildet, wobei der aromatische Ring einen Substituenten $NO_2$ tragen kann.

**2.** Kontrastmittel der Formel (II) nach Anspruch 1, **dadurch gekennzeichnet, dass:**

- $R^6$ für eine β-Galactosylgruppe, eine β-Glucuronylgruppe, eine L-Prolylgruppe, eine L-Leucylgruppe, eine $-COC_5H_{11}$-Gruppe, eine Gruppe vom Typ α,β-Dihydroxyceton

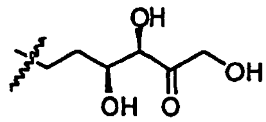

oder eine Phosphorylgruppe steht;
- die X-Gruppen unabhängig voneinander für ein Wasserstoffatom, ein Fluoratom oder eine $NO_2$-Grupp stehen.

**3.** Kontrastmittel nach Anspruch 1, das der Formel (Ia) entspricht, **dadurch gekennzeichnet, dass** die $R^i$-Gruppen unabhängig voneinander für ein Wasserstoffatom, eine elektronenziehende Gruppe, ausgewählt aus den $-COOR^7$-Gruppen, worin $R^7$ für eine Alkylgruppe steht, die 1 bis 4 Kohlenstoffatome aufweist, die $-NO_2$-Gruppe, ein Halogenatom und die $-CH_2-COO^-$-Gruppe stehen, oder eine der drei $R^i$-Gruppen für eine - CH(OH)CH(COOH) $NH_2$-Gruppe steht, wobei die verbleibenden $R^i$ dann so sind, wie oben definiert.

**4.** Kontrastmittel nach Anspruch 3, das der Formel Ia entspricht, **dadurch gekennzeichnet, dass** $R^5$ der Formel -E-$R^6$ entspricht, worin E eine Spacergruppe ist, die in der Lage ist, im Fall der Trennung der Bindung E-$R^6$ in sich selbst zusammenzufallen und $R^6$ eine Gruppe ist, die in der Lage ist, mit einer chemischen Verbindung oder einer biochemischen Verbindung zu reagieren, indem sie eine Abfolge von Trennungen auslöst, die die Elimination von Pyridin-2-carbaldehyd herbeiführt.

**5.** Kontrastmittel nach Anspruch 4, **dadurch gekennzeichnet, dass:**

- $R^6$ für eine Gruppe steht, die eine Spaltung durch eine Glycosidase erfahren kann, oder eine Aminoacylgruppe, die eine Spaltung durch eine Aminopeptidase erfahren kann, oder eine Acylgruppe, die eine Spaltung durch eine Esterase oder eine Lipase erfahren kann, oder eine Gruppe, die eine Retro-Aldol-Umwandlung durch eine natürliche Aldolase erfahren kann, oder eine Gruppe, die eine Umwandlung durch eine Phosphatase erfahren kann, zum Beispiel eine Phosphorylgruppe;
- E eine $-O-CO-(CR'_2)_n-Y-$-Gruppe ist, worin die R'-Gruppen unabhängig voneinander für ein Wasserstoffatom, eine Methylgruppe stehen, oder zwei R', die von zwei benachbarten Kohlenstoffatomen getragen werden, zusammen einen aliphatischen oder aromatischen 5- oder 6-gliedrigen Ring bilden, der von den beiden benachbarten Kohlenstoffatomen getragen wird;
- n ein ganze Zahl gleich 3 oder 4 ist;
- Y ein Heteroatom ist, das aus einem Sauerstoffatom, einem Stickstoffatom oder einem Schwefelatom ausgewählt ist.

**6.** Kontrastmittel nach Anspruch 5, **dadurch gekennzeichnet, dass** $R^6$ für eine β-Galactosylgruppe, eine β-Glucuronylgruppe, eine L-Prolylgruppe, eine L-Leucylgruppe, eine $-C0C_5H_{11}$-Gruppe, eine Gruppe vom Typ α,β-Dihydroxyceton

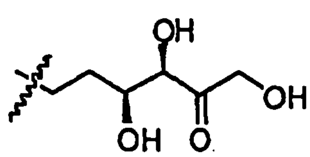

oder eine Phosphorylgruppe steht;

**7.** Kontrastmittel nach Anspruch 5 oder 6, **dadurch gekennzeichnet, dass** der aromatische 5- oder 6-gliedrige Ring aus den Phenyl-, Pyridyl-, Pyrimidinyl-, Pyrazinyl-, Indoyl-, Indazolyl-, Furyl- und Thienylgruppen ausgewählt ist, wobei die Gruppen verschiedene Substituenten tragen können, die aus den Methyl-, Chloro-, Fluoro-, Nitro-, Methoxy-, Carboxy- und Acetamidogruppen ausgewählt sind, und der aliphatische 5- oder 6-gliedrige Ring eine Cyclohexylgruppe oder eine Cyclopentylgruppe ist.

**8.** Kontrastmittel nach einem der Ansprüche 5 bis 7, **dadurch gekennzeichnet, dass** E einer der folgenden Formeln entspricht:

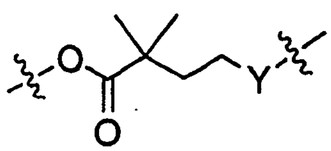

E1

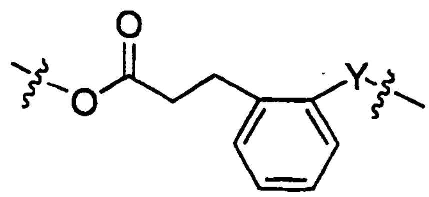

E2

**9.** Kontrastmittel nach Anspruch 1 oder 3, das der Formel Ia entspricht, **dadurch gekennzeichnet, dass:**

- $R^5$ der Formel-$C_6X_4$-Y-$R^6$ entspricht, worin ($C_6X_4$-Y) eine Spacergruppe ist, die in der Lage ist, im Fall einer Trennung der Verbindung -($C_6X_4$-Y)-$R^6$ in sich selbst zusammenzufallen, und
- $R^6$ eine Gruppe ist, die in der Lage ist, mit einer chemischen Verbindung oder mit einer biochemischen Verbindung zu reagieren, indem sie eine Abfolge von Trennungen auslöst, die die Elimination eines Chinonmethids herbeiführt.

**10.** Kontrastmittel nach Anspruch 9, **dadurch gekennzeichnet, dass:**

- Y ein Heteroatom ist, das aus einem Sauerstoffatom, einem Stickstoffatom oder einem Schwefelatom ausgewählt ist;
- X eine elektronenziehende Gruppe ist, und
- $R^6$ für eine Gruppe steht, die eine Spaltung durch eine Glycosidase erfahren kann, oder eine Aminoacylgruppe, die eine Spaltung durch eine Aminopeptidase erfahren kann, oder eine Acylgruppe, die eine Spaltung durch eine Esterase oder eine Lipase erfahren kann, oder eine Gruppe, die eine Retro-Aldol-Umwandlung durch eine natürliche Aldolase erfahren kann, oder eine Gruppe, die eine Umwandlung durch eine Phosphatase erfahren kann, zum Beispiel eine Phosphorylgruppe.

**11.** Kontrastmittel nach Anspruch 10, **dadurch gekennzeichnet, dass:**

- $R^6$ für eine β-Galactosylgruppe, eine β-Glucuronylgruppe, eine L-Prolylgruppe, eine L-Leucylgruppe, eine -$COC_5H_{11}$-Gruppe, eine Gruppe vom Typ α,β-Dihydroxyceton

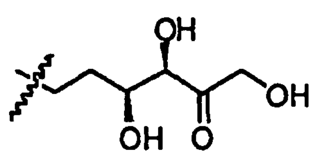

oder eine Phosphorylgruppe steht;
- die X-Gruppen unabhängig voneinander für ein Wasserstoffatom, ein Fluoratom oder eine $NO_2$-Gruppe stehen.

**12.** Kontrastmittel nach einem der Ansprüche 9 bis 11, **dadurch gekennzeichnet, dass** ($C_6X_4$-Y) einer der folgenden Formeln entspricht:

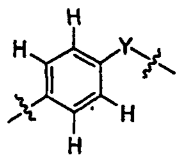 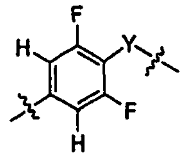 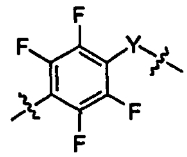 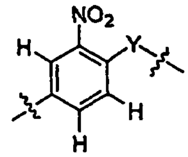

**13.** Kontrastmittel nach Anspruch 1, das der Formel Ib entspricht, **dadurch gekennzeichnet, dass** eine der drei $R^i$-Gruppen für eine $-CH(OH)CH(COOH)NH_2$-Gruppe steht und die verbleibenden $R^i$-Gruppen unabhängig voneinander für ein Wasserstoffatom, ein Halogenatom oder eine Gruppe, die aus den $-COOR^7$-Gruppen ausgewählt ist, worin $R^7$ für eine Alkylgruppe, die 1 bis 4 Kohlenstoffatome aufweist, die $-NO_2$-Gruppe, die -CHO-Gruppe stehen können.

**14.** Kontrastmittel nach Anspruch 1 oder 2, das der Formel (II) entspricht, **dadurch gekennzeichnet, dass** $(C_6X_4$-Y) einer der folgenden Formeln entspricht:

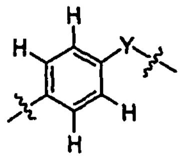 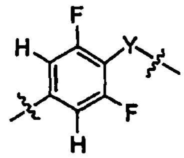 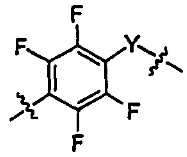 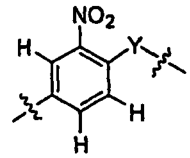

**15.** Pharmazeutische Zusammensetzung, **dadurch gekennzeichnet, dass** sie ein Kontrastmittel nach einem der Ansprüche 1 bis 14 umfasst.

**16.** Verwendung eines Kontrastmittels nach einem der Ansprüche 1 bis 14 zur Herstellung einer pharmazeutischen Zusammensetzung, die der Diagnose mittels Magnetresonanztomographie dient.

**17.** Pharmazeutische Zusammensetzung nach Anspruch 15, die der Bestimmung der Gewebeverteilung von β-Galactosidase oder β-Glucuronidase dient, **dadurch gekennzeichnet, dass** das Kontrastmittel so ist, wie in einem der Ansprüche 4 oder 9 definiert, wobei $R^6$ jeweils für eine β-Galactosylgruppe oder eine β-Glucuronylgruppe steht.

**18.** Pharmazeutische Zusammensetzung nach Anspruch 15, die der Bestimmung der Gewebeverteilung von Aminopeptidasen, Lipase, Transaldolasen und Phosphatasen dient, **dadurch gekennzeichnet, dass** das Kontrastmittel so ist, wie in einem der Ansprüche 4 oder 9 definiert, wobei $R^6$ für eine *L*-Leucylgruppe, eine $-COC_5H_{11}$-Gruppe, eine Gruppe vom Typ α,β-Dihydroxyaceton

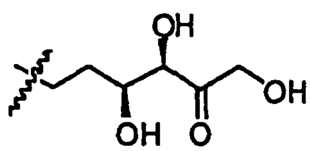,

eine Prolylgruppe oder eine Phosphorylgruppe steht.

**19.** Pharmazeutische Zusammensetzung nach Anspruch 15, die der Bestimmung der Gewebeverteilung von *L*-Threoninaldolase dient, **dadurch gekennzeichnet, dass** das Kontrastmittel so ist, wie in Anspruch 13 definiert.

**20.** Verbindung der Formel (4),

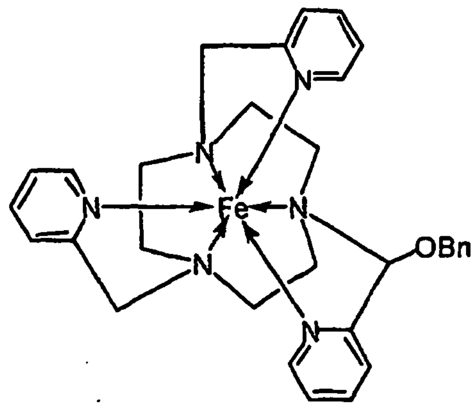

(4)

die als Synthesezwischenprodukt nützlich ist.

**21.** Verfahren zur Herstellung eines Komplexes der Formel (Ia), worin $R^5$ und $R^i$ sind, wie in einem der Ansprüche 4 bis 8 definiert, **dadurch gekennzeichnet, dass** eine Kopplung einer Verbindung (4) nach Anspruch 20, die zuvor hydriert wurde, mit einem Acylchlorid der Formel $Cl\text{-}CO\text{-}(CR'_2)_n\text{-}Y\text{-}R^6$ in Gegenwart einer Base durchgeführt wird, um die Verbindung zu erhalten, worin die Gruppe $R^6$ optional in geschützter Form vorliegt, optional gefolgt von einer Entschützung, um den Komplex der Formel (Ia-1) zu erhalten.

**22.** Verfahren zur Herstellung eines Komplexes der Formel (Ia-1), worin $R^5$ eine $\text{-O-CO-C}(CH_3)_2\text{-}(CH_2)_2\text{-}OR^6$-Gruppe ist, $R^6$ eine penta-acetylierte β-Galactosylgruppe ist und die anderen Substituenten $R^i$ und $R^4$ Wasserstoffatome sind, **dadurch gekennzeichnet, dass** es einen Schritt umfasst, der darin besteht, eine Verbindung (4) nach Anspruch 21, die zuvor hydriert wurde, mit einer Verbindung der folgenden Formel (6):

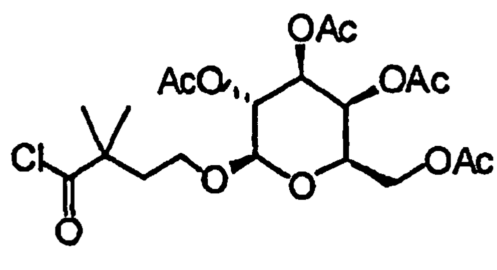

(6)

in Gegenwart einer Base umzusetzen, um den Komplex zu erhalten, worin die Galactosylgruppe in geschützter Form vorliegt, gefolgt von einer Entschützung, um den Komplex der Formel (Ia-1) zu erhalten.

**23.** Verbindung der Formel (9)

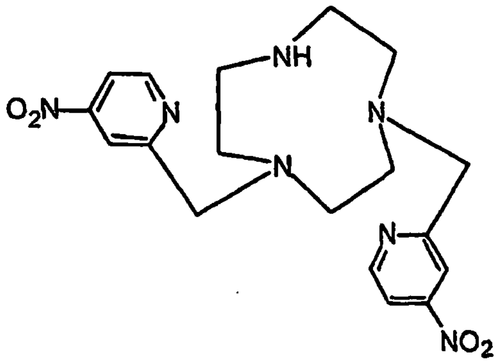

(9)

die als Synthesezwischenprodukt nützlich ist.

**24.** Verfahren zur Herstellung eines Komplexes der Formel (Ia-2), worin $R^5$ eine $C_6H_4$-O-$R^6$-Gruppe ist, wobei $R^6$ eine penta-acetylierte β-Galactosylgruppe ist und die anderen Substituenten $R^i$ und $R^4$ Wasserstoffatome sind, **dadurch gekennzeichnet, dass** es einen Schritt umfasst, der darin besteht, eine Verbindung der folgenden Formel (2'):

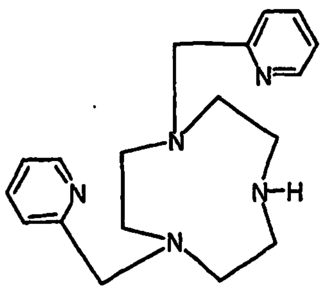

(2')

mit einer Verbindung der folgenden Formel (5'):

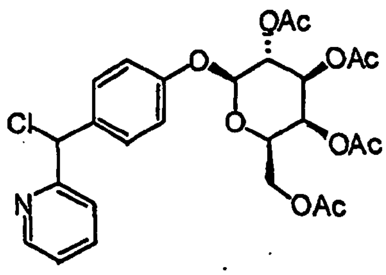

(5')

umzusetzen, um die Verbindung der folgenden Formel (6') zu erhalten, worin die Galactosylgruppe in geschützter Form vorliegt,

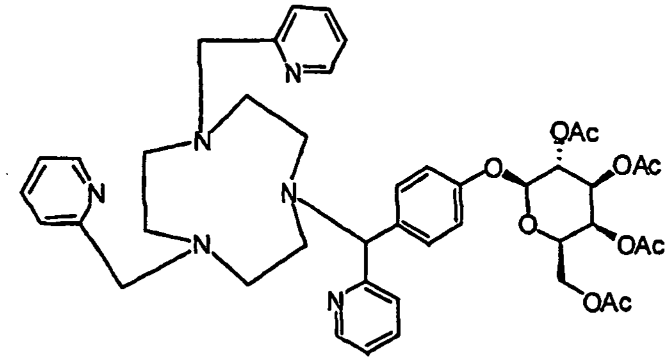

(6')

gefolgt von einem Schritt der Entschützung und einem Schritt der Behandlung mit $Fe(BF_4)_2$, um den Komplex der Formel (Ia-2) zu erhalten.

**25.** Verfahren zur Herstellung eines Komplexes der Formel (Ib), worin $R^5$ ein Wasserstoffatom ist und einer der Substituenten $R^i$ eine $CH(OH)CH(COOH)NH_2$-Gruppe ist, **dadurch gekennzeichnet, dass** es einen Schritt umfasst, der darin besteht, eine Verbindung der Formel (9) nach Anspruch 23 mit einer Verbindung der folgenden Formel (8):

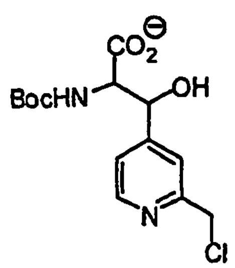

(8)

umzusetzen, gefolgt von einem Schritt der Entschützung der primären Amingruppe und einer Umwandlung in einen Eisenkomplex, um den Komplex der Formel (Ib) zu erhalten.

**26.** Verfahren zur Herstellung eines Komplexes der Formel (II), worin $R^5$ eine-$C_6H_4$-0-β-Galactosylgruppe ist, wobei die beiden Stickstoffatome und die Z-Gruppe zusammen eine Benzotriazoleinheit bilden, und $R^1$, $R^2$ und $R^4$ für Wasserstoffatome stehen,
**dadurch gekennzeichnet, dass** es einen Schritt umfasst, der darin besteht, eine Verbindung der folgenden Formel (1''):

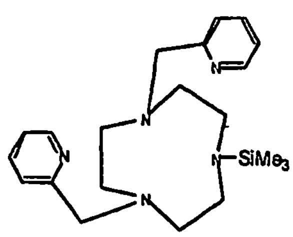

(1")

mit einer Verbindung der folgenden Formel (2"):

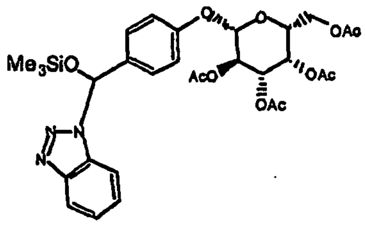

(2")

umzusetzen, um die Verbindung der folgenden Formel (3 ") zu erhalten, worin die Galactosylgruppe in geschützter Form vorliegt,

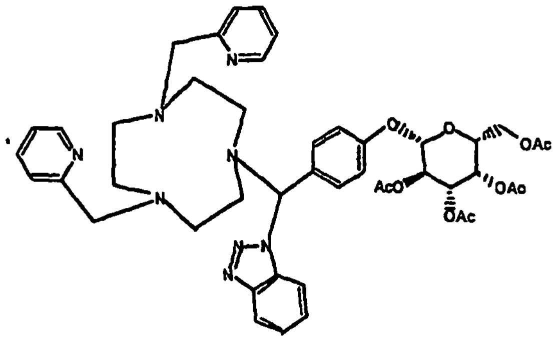

(3")

gefolgt von einem Schritt der Behandlung mit $Fe(BF_4)_2$, gefolgt von einem Schritt der Entschützung, um den Komplex der Formel (II) zu erhalten.

**27.** Verwendung eines Kontrastmittels nach Anspruch 16, das der Formel (Ia) entspricht, worin $R^5$ für eine -E-$R^6$-Gruppe oder eine ($C_6X_4$-Y-$R^6$)-Gruppe steht, wobei $R^6$ jeweils für eine β-Galactosylgruppe oder eine β-Glucuronylgruppe steht, und die Gruppen E, ($C_6X_4$-Y), $R^4$ und $R^i$ so sind, wie in einem der Ansprüche 3 bis 12 definiert, zur Herstellung einer pharmazeutischen Zusammensetzung, die der Bestimmung der Gewebeverteilurig von β-Galactosidase oder β-Glucuronidase dient.

**28.** Verwendung eines Kontrastmittels nach Anspruch 16, das der Formel (Ia) entspricht, worin $R^5$ für eine E-$R^6$-Gruppe oder eine ($C_6X_4$-Y-$R^6$)-Gruppe steht, wobei $R^6$ für eine L-Leucylgruppe, eine -$COC_5H_{11}$,-Gruppe, eine Gruppe vom Typ α,β-Dihydroxyaceton

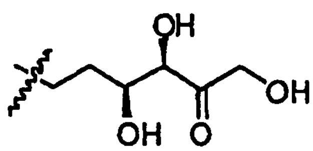

,

eine Prolylgruppe oder eine Phosphorylgruppe steht, und die Gruppen E, ($C_6X_4$-Y), $R^4$ und $R^i$ so sind, wie in einem der Ansprüche 3 bis 12 definiert, zur Herstellung einer pharmazeutischen Zusammensetzung, die der Bestimmung der Gewebeverteilung von Aminopeptidase, Lipase, Transaldolase oder Phosphatase dient.

**29.** Verwendung eines Kontrastmittels nach Anspruch 16, das der Formel (Ib) nach Anspruch 13 entspricht, zur Herstellung einer pharmazeutischen Zusammensetzung, die der Bestimmung der Gewebeverteilung von L-Threoninaldolase dient.

**30.** Verfahren der Magnetresonanztomographie einer Zelle oder eines Gewebes, dem ein Kontrastmittel nach einem der Ansprüche 1 bis 14 appliziert wurde.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5707605 A
- WO 9921592 A


### Littérature non-brevet citée dans la description


- **Meade, T. J. et al.** In vivo visualization of gene expression using magnetic resonance imaging. *Nature Biotechnology,* 2000, vol. 18, 321-325
- **Wieghardt et al.** *Inorg. Chem.,* 1986, vol. 25, 4877
- **Spiccia et al.** *Inorg. Chim. Acta,* 1998, vol. 279, 192
- Solution NMR of Paramagnetic Molecules. **I. Bertini ; C. Luchinat ; G. Parigi.** Current Methods in Inorganic Chemistry. Elsevier, 2001, vol. 2
- **R. B. Lauffer.** *Chem. Rev.,* 1987, vol. 87, 901
- **L.A. Carpino.** *J. Org. Chem.,* 1989, vol. 54, 3303
- **E. Gonzalez-Garcia et al.** *Chem. Eur. J.,* 2003, vol. 9, 893-899
- **Spiccia et al.** *Inorg. Chem.,* 1994, vol. 33, 4663
- **Ugalde-Saldivar et al.** *J. Chem. Soc. Dalton Trans.,* 2001, 3099-3107
- **Bishop et al.** *Tetrahedron,* 2000, 4629-4638
- **P.B. Rasmussen et al.** *Bull. Soc. Chem. Fr.,* 1985, vol. II, 62-64
- *Inorg. Chem.,* 1989, vol. 28, 4022-4026
- **A. Bourry et al.** *J. Heterocylic Chem.,* 2002, vol. 39, 109-118